# EUROPEAN PATENT APPLICATION

(11) **EP 4 227 310 A1**
(43) Date of publication of application: **16.08.2023**
(21) Application number: 21879305.7
(22) Date of filing: 09.10.2021
(51) Int. Cl.: C07D 491/22, A61K 31/4375, A61K 47/68, A61P 35/00

(54) **CAMPTOTHECIN DERIVATIVE AND LIGAND-DRUG CONJUGATE THEREOF**

(30) Priority: 12.10.2020 CN 202011086123
(71) Applicant: Sichuan Baili Pharmaceutical Co. Ltd., Chengdu, Sichuan 611130 (CN); Baili-Bio (Chengdu) Pharmaceutical Co., Ltd., ChengDu, Sichuan 611130 (CN)
(72) Inventor: ZHU, Yi, Chengdu, Sichuan 611130 (CN); WAN, Weili, Chengdu, Sichuan 611130 (CN); ZHU, Guili, Chengdu, Sichuan 611130 (CN); YU, Tianzi, Chengdu, Sichuan 611130 (CN); ZHUO, Shi, Chengdu, Sichuan 611130 (CN); YANG, Xiujuan, Chengdu, Sichuan 611130 (CN)
(74) Representative: Haile, Alison Victoria
(86) International application number: PCT/CN2021/122823
(87) International publication number: WO 2022/078260

(57) **Abstract**

The present invention discloses a camptothecin derivative and its ligand-drug conjugate thereof. The present invention discloses a superior anti-tumor camptothecin ADC drug with higher safety and efficacy to better meet clinical needs.

## Description

### Technical Field

The present invention relates to a camptothecin derivative and a ligand-drug conjugate.

### Background

Ligand-drug conjugates (ADC) are a novel class of targeted drugs that generally consists of three parts: an antibody or antibody-like ligand, a small molecule drug, and a linking unit that links the ligand and the drug. Antibody-drug conjugates use the specific recognition of antigens by antibodies to transport drug molecules to the vicinity of target cells and effectively release drug molecules to achieve therapeutic purposes. In August 2011, the U.S. Food and Drug Administration (FDA) approved ADCETRIS^{®}, a new ADC drug developed by Seattle Genetics for the treatment of Hodgkin lymphoma and recurrent degenerative large cell lymphoma (ALCL), and clinical application has demonstrated the safety and efficacy of such drugs.

Camptothecines are small molecule compounds known to exhibit antitumour effects as inhibitors of DNA topoisomerase I, including irinotecan, exatecan, SN38 and others. Many camptothecins and analogues have been used widely in clinical practice for the main indications of bone cancer, prostate cancer, breast cancer, pancreatic cancer and so on. Unlike irinotecan, which is currently in clinical use, exatecan does not require enzymes for activation. In addition, compared to SN-38, which is the active agent of irinotecan, and topotecan, which is also in clinical use, exatecan's topoisomerase I inhibitory activity is stronger, and has stronger cytotoxic activity to a variety of cancer cells *in vitro.* In particular, it has also shown an effect on cancer cells that show resistance to SN-38 through the expression of P-glycoprotein. Exatecan has not been successfully marketed as a stand-alone chemotherapeutic agent, presumably due to its higher cellular activity, resulting in a narrow therapeutic window.

The advantages of antibody-drug conjugate (ADC) drugs include increased water solubility, improved targeting, specific antibody-antigen binding, carrying the drug around target cells, effectively killing of tumor cells by releasing the drug in the vicinity of the target cells, and reduced toxic side effects. Camptothecins and analogue drugs have promising applications in ADC drugs, and the antibody-conjugated drug trastuzumab deruxtecan (trade name ENHERTU^{®}) with exatecan as the toxin has been approved for marketing by the US FDA on December 20, 2019, as the first marketed camptothecin-type ADC drug, it has well proved the drug-making ability and application prospect of this type of drug in the field of ADC.

ENHERTU^{®} is an ADC drug targeting HER2, in which 8 exatecan derivatives (DAR=8) are linked to a single antibody. At the same time, its original research company Daiichi Sankyo also realized that it is necessary to reduce the DAR value for safety reasons. DS-1062, its published ADC used in clinical practice, has a reduced DAR value of 4. DAR value decrease may lead to a decrease in the therapeutic effect. Thus, it is necessary to develop safer camptothecin derivatives.

In ADC drugs, in order to ensure safety, the DAR value of the drug may be lowered, resulting in a reduction in the therapeutic effect. Drug solubility is one of the important factors affecting the DAR value, and it is also the main factor affecting the bystander effect of ADC drugs. Although camptothecin drugs have been widely used in clinical practice, their poor water solubility is still a problem waiting to be solved. For drugs with poor water solubility, increasing the water solubility of the drug can improve the physical properties of the drug and change its metabolism in the body, thereby improving the efficacy of the drug, strengthening the therapeutic effect, reducing the dosage of the drug, and reducing the cost. Poor water solubility will lead to increased aggregation of ADC, facilitating endocytosis by normal cells, resulting in toxic side effects or promoting metabolism and shortening the halflife.

While conducting research, the inventors unexpectedly discovered that the amino chiral isomer compound of exatecan has significantly better water solubility than that of exatecan, and its synthesized derivatives also have better water solubility and can be synthesized into better anti-tumor camptothecin ADC drugs, so that they have higher safety and effectiveness, and better meet clinical needs.

### Summary

The present invention provides a camptothecin derivative, represented by Formula D, or its tautomer, mesomer, racemate, enantiomer, diastereoisomer or a mixture thereof, or pharmaceutically acceptable salt or solvate thereof; 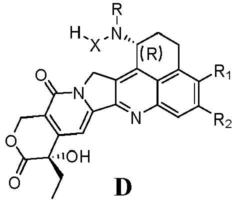 wherein:
the chiral carbon atom connected to the 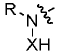 group has an absolute chirality of R configuration;
R is selected from the group consisting of hydrogen, alkyl, substituted alkyl, deuterated alkyl, cycloalkylalkyl, alkoxy alkyl, aryl, substituted aryl and heteroaryl;
R₁ is selected from the group consisting of hydrogen, deuterium, halogen, alkyl, substituted alkyl, deuterated alkyl, cycloalkylalkyl, alkoxy alkyl, carboxyl, heterocyclic, aryl, substituted aryl and heteroaryl;
R₂ is selected from the group consisting of hydrogen, deuterium, halogen, alkyl, substituted alkyl, deuterated alkyl, cycloalkylalkyl, alkoxy alkyl, carboxyl, heterocyclic, aryl, substituted aryl and heteroaryl;
X is selected from -C(O)-CRₐR_{b}-(CR₃R₄)ₘ-O-, -C(O)-CRₐR_{b}-(CR₃R₄)ₘ-NH-, and - C(O)-CRₐR_{b}-(CR₃R₄)ₘ-S-;
Rₐ is selected from the group consisting of hydrogen, deuterium, halogen, alkyl, deuterated alkyl, halogenated alkyl, cycloalkylalkyl, alkoxy alkyl, heterocyclic, aryl, substituted aryl and heteroaryl;
R_{b} is selected from the group consisting of hydrogen, deuterium, halogen, alkyl, deuterated alkyl, halogenated alkyl, cycloalkylalkyl, alkoxy alkyl, heterocyclic, aryl, substituted aryl and heteroaryl;
alternatively, Rₐ, R_{b} and their connected carbon atoms form a C₃₋₆ cycloalkyl group, a cycloalkylalkyl or a heterocyclic group;
R₃, R₄ are identical or different, and are independently selected from the group consisting of hydrogen, deuterium, halogen, alkyl, haloalkyl, deuterated alkyl, alkoxy, hydroxyl, amino, cyano, nitro, hydroxyalkyl, cycloalkyl and heterocyclic;
alternatively, R₃, R₄ and their connected carbon atoms form a C₃₋₆ cycloalkyl group, a cycloalkylalkyl or a heterocyclic group; and
m is an integer selected from 0-4.

In a preferred embodiment, R₁ is selected from C₁₋₃ alkyl groups.

In a preferred embodiment, R₂ is selected from C₁₋₃ alkyl groups and Fluoride.

Disclosed is a camptothecin derivative having the structure shown in the following Formula **D₁:** 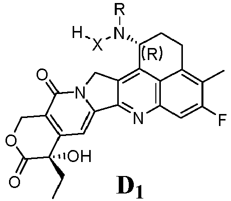 wherein:
the chiral carbon atom connected to the 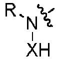 group has an absolute chirality of R configuration;;
R is selected from the group consisting of hydrogen, alkyl, substituted alkyl, deuterated alkyl, cycloalkylalkyl, alkoxy alkyl, aryl, substituted aryl and heteroaryl;
X is selected from -C(O)-CRₐR_{b}-(CR₃R₄)ₘ-O-, -C(O)-CRₐR_{b}-(CR₃R₄)ₘ-NH-, and - C(O)-CRₐR_{b}-(CR₃R₄)ₘ-S-;
Rₐ is selected from the group consisting of hydrogen, deuterium, halogen, alkyl, deuterated alkyl, halogenated alkyl, cycloalkylalkyl, alkoxy alkyl, heterocyclic, aryl, substituted aryl and heteroaryl;
R_{b} is selected from the group consisting of hydrogen, deuterium, halogen, alkyl, deuterated alkyl, halogenated alkyl, cycloalkylalkyl, alkoxy alkyl, heterocyclic, aryl, substituted aryl and heteroaryl;
alternatively, Rₐ, R_{b} and their connected carbon atoms form a C₃₋₆ cycloalkyl group, a cycloalkylalkyl or a heterocyclic group;
R₃, R₄ are identical or different, and are independently selected from the group consisting of hydrogen, deuterium, halogen, alkyl, haloalkyl, deuterated alkyl, alkoxy, hydroxyl, amino, cyano, nitro, hydroxyalkyl, cycloalkyl and heterocyclic;
alternatively, R₃, R₄ and their connected carbon atoms form a C₃₋₆ cycloalkyl group, a cycloalkylalkyl or a heterocyclic group;
m is an integer selected from 0-4.

In a preferred embodiment, Ris selected from C₁₋₃ alkyl groups.

In a preferred embodiment, The X is selected from the following structures without limitation: 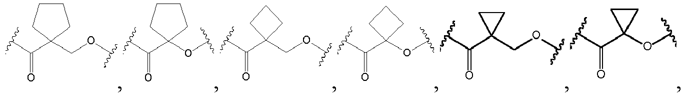 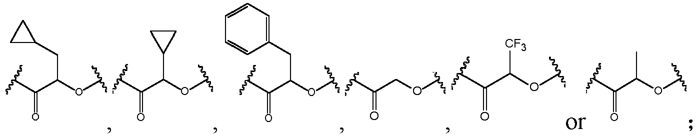 wherein the left wavy lines are connected to the camptothecin derivative, and the right wavy lines are connected to a linking unit.

In a preferred embodiment, the camptothecin derivative is selected from the following compounds without restriction. 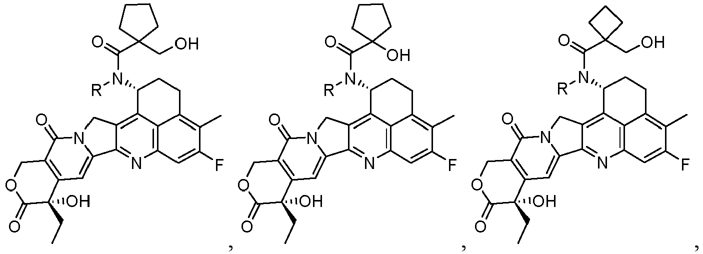 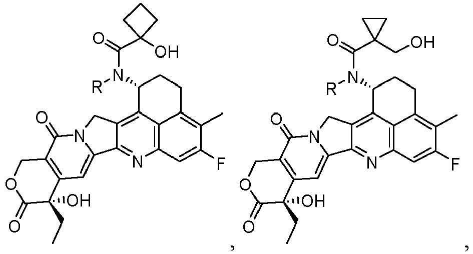 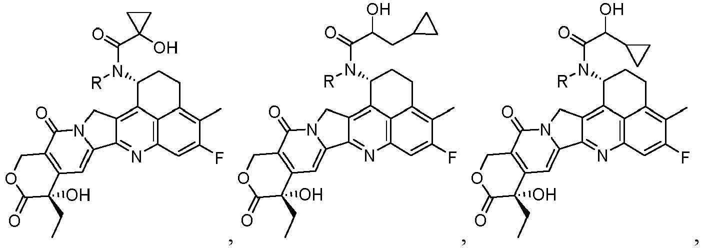 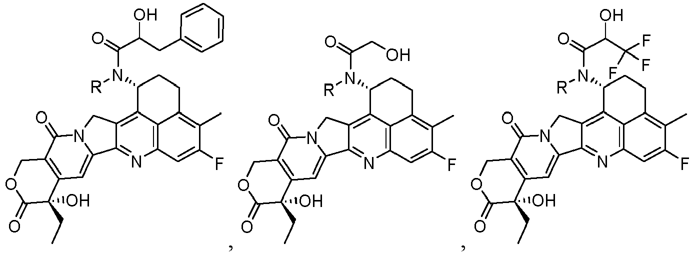 or 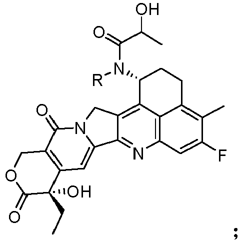 wherein R is selected from the group consisting of hydrogen, deuterium, halogen, alkyl, substituted alkyl, deuterated alkyl, cycloalkylalkyl, alkoxy alkyl groups, aryl, substituted aryl and heteroaryl.

In a preferred embodiment, the camptothecin derivative is selected from the following compounds without restriction:

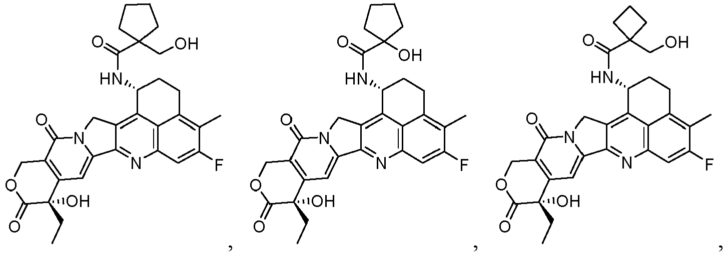

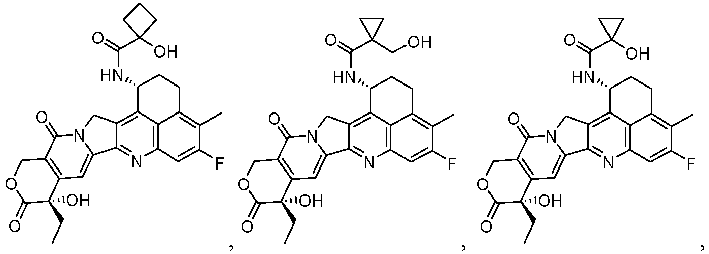

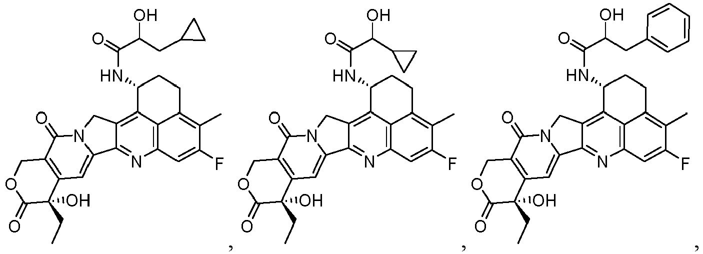

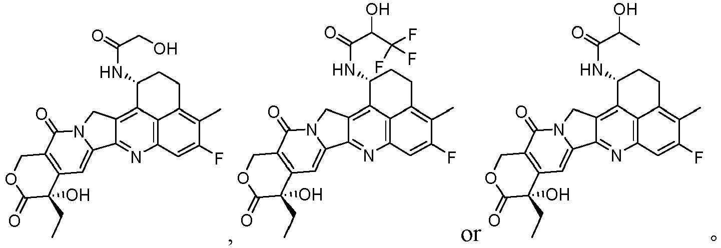

The present invention also provides a linker-drug conjugate comprising a camptothecin derivative, or a pharmaceutically acceptable salt or solvate thereof, or its tautomer, mesomer, racemate, enantiomer, diastereoisomer or a mixture thereof: 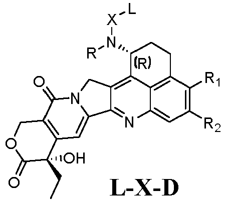 wherein:
the chiral carbon atom connected to the 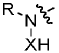 group has an absolute chirality of R configuration;
R is selected from the group consisting of hydrogen, alkyl, substituted alkyl, deuterated alkyl, cycloalkylalkyl, alkoxy alkyl, aryl, substituted aryl and heteroaryl;
R₁ is selected from the group consisting of hydrogen, deuterium, halogen, alkyl, substituted alkyl, deuterated alkyl, cycloalkylalkyl, alkoxy alkyl, carboxyl, heterocyclic, aryl, substituted aryl and heteroaryl;
R₂ is selected from the group consisting of hydrogen, deuterium, halogen, alkyl, substituted alkyl, deuterated alkyl, cycloalkylalkyl, alkoxy alkyl, carboxyl, heterocyclic, aryl, substituted aryl and heteroaryl;
X is selected from -C(O)-CRₐR_{b}-(CR₃R₄)ₘ-O-, -C(O)-CRₐR_{b}-(CR₃R₄)ₘ-NH-, or - C(O)-CRₐR_{b}-(CR₃R₄)ₘ-S-;
Rₐ is selected from the group consisting of hydrogen, deuterium, halogen, alkyl, deuterated alkyl, halogenated alkyl, cycloalkylalkyl, alkoxy alkyl, heterocyclic, aryl, substituted aryl and heteroaryl;
R_{b} is selected from the group consisting of hydrogen, deuterium, halogen, alkyl, deuterated alkyl, halogenated alkyl, cycloalkylalkyl, alkoxy alkyl, heterocyclic, aryl, substituted aryl and heteroaryl;
alternatively, Rₐ, R_{b} and their connected carbon atoms form a C₃₋₆ cycloalkyl group, a cycloalkylalkyl group or a heterocyclic group;
R₃, R₄ are identical or different, and are independently selected from the group consisting of hydrogen, deuterium, halogen, alkyl, haloalkyl, deuterated alkyl, alkoxy, hydroxyl, amino, cyano, nitro, hydroxyalkyl, cycloalkyl and heterocyclic;
alternatively, R₃, R₄ and their connected carbon atoms form a C₃₋₆ cycloalkyl group, a cycloalkylalkyl group or a heterocyclic group;
m is an integer selected from 0-4; and
L comprises -L₁-L₂-L₃-L₄-.

In a preferred embodiment, L is -L₁-L₂-L₃-L₄- and the L₁ is connected to a ligand Ab and the L₄ is connected to the X.

In a preferred embodiment, the L₁ is selected from the following structures without restriction:

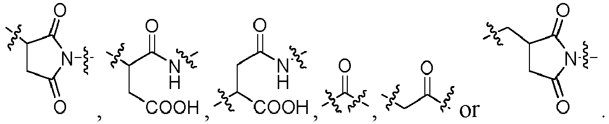

In a preferred embodiment, L₂ is selected from -NC(R₅R₆)C(O), -NR₇(CH₂)ₒC(O)-, - NR₇(CH₂CH₂O)ₒCH₂C(O)-, -S(CH₂)ₚC(O)- or a chemical bond, wherein o is an integer from 0-20, p is an integer from 0-20;
R₅ and R₆ are identical or different, and independently selected from the group consisting of hydrogen, deuterium, alkyl, substituted alkyl, deuterated alkyl, heteroalkyl, carboxyl, amino, and substituted amino;
R₇ is selected from the group consisting of hydrogen, deuterium, halogen, alkyl, substituted alkyl, deuterated alkyl, cycloalkylalkyl, alkoxy alkyl, aryl, substituted aryl and heteroaryl;
L₁ and L₂ use a common N atom.

In a preferred embodiment, wherein L₃ comprises a peptide residue made from free amino acids, wherein the amino acids may be further substituted by one or more substituents selected from the group consisting of deuterium, halogen, hydroxyl, cyano, amino, nitro, carboxyl, alkyl, substituted alkyl, alkoxy, cycloalkyl or substituted cycloalkyl. In a preferred embodiment, the peptide residue comprises one, two or more amino acids selected from the group consisting of phenylalanine (F), glycine (G), valine (V), lysine (K), citrulline, serine (S), glutamic acid (E), and aspartic acid (D).

In a preferred embodiment, L₄ is selected from: NR₈(CR₉R₁₀)_{q}-, -C(O)NR₈-, - C(O)NR₈(CH₂)_{q}- or a chemical bond, q is selected from integers 0-6;
R₈, R₉ and R₁₀ are identical or different, and independently selected from the group consisting of hydrogen, deuterium, halogen, alkyl, substituted alkyl, deuterated alkyl, cycloalkylalkyl, alkoxy alkyl, heterocyclic, aryl, substituted aryl and heteroaryl.

In a preferred embodiment, wherein the linker L is selected from the following structures without restriction, 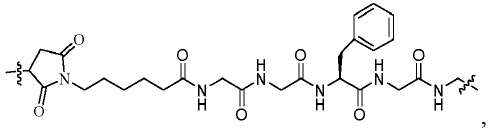 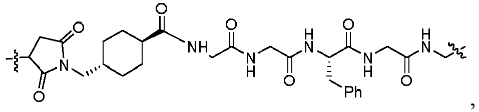 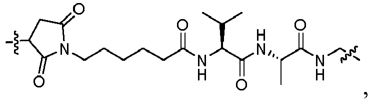 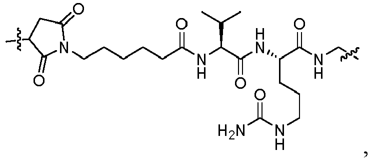 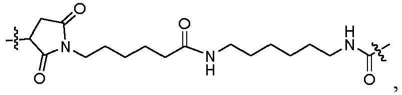 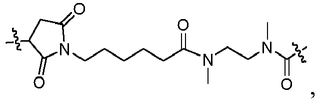 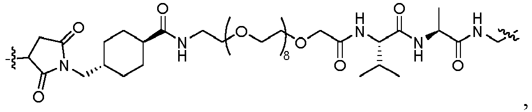 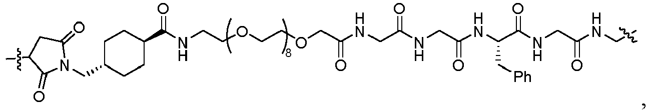 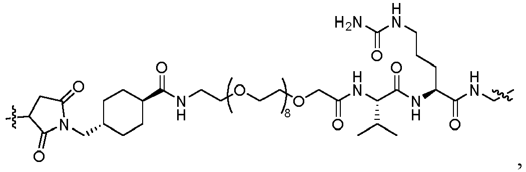 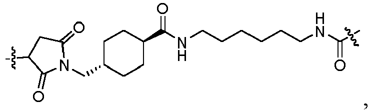 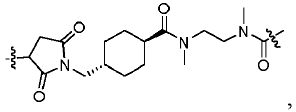 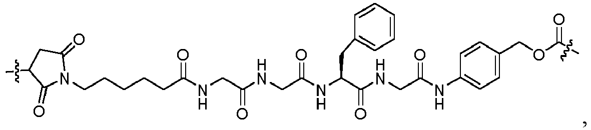 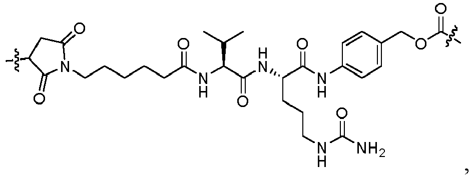 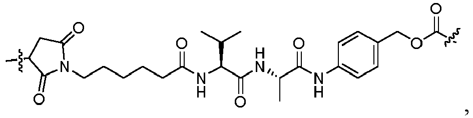 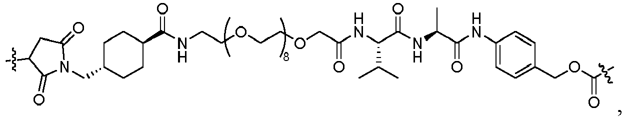 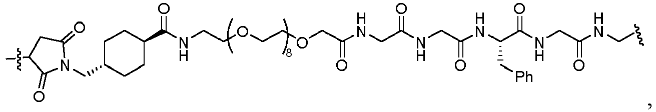 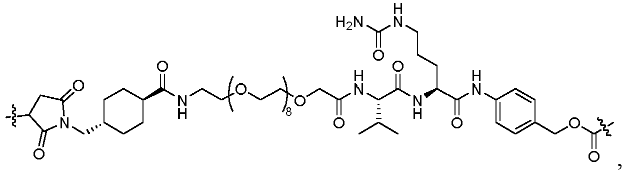 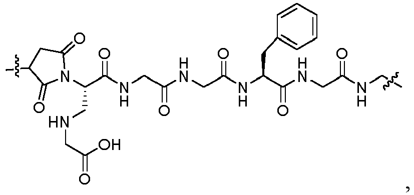 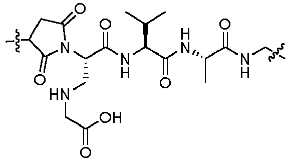 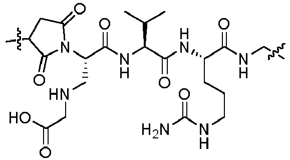 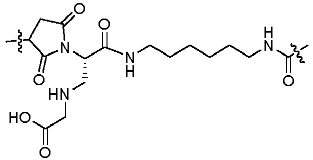 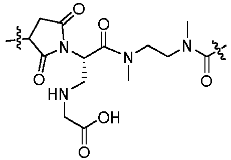 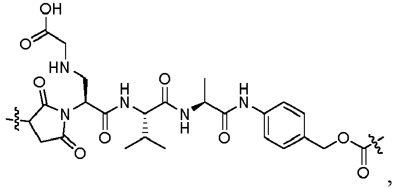 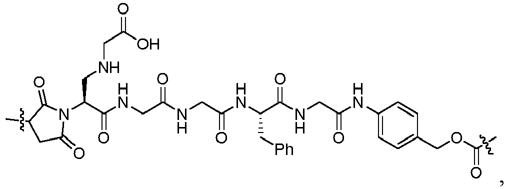 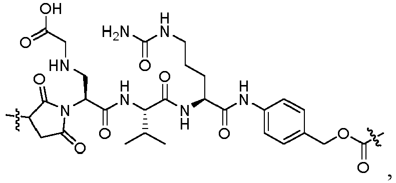 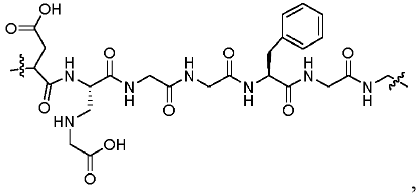 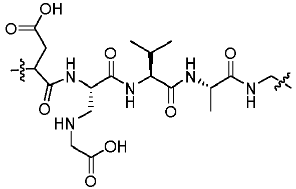 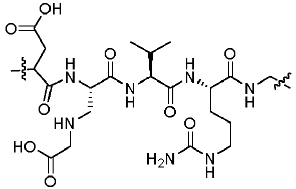 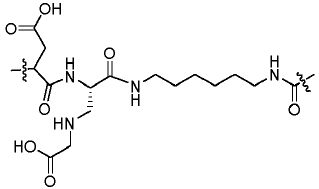 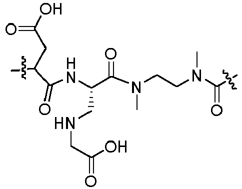 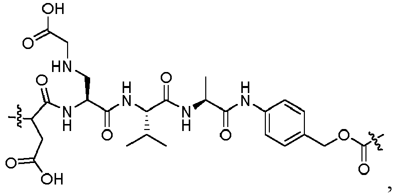 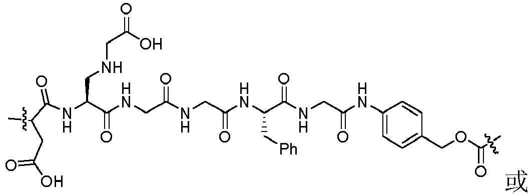 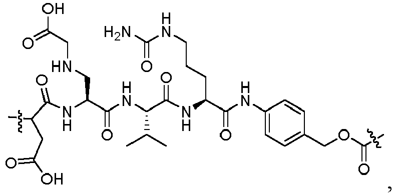 wherein the left wavy lines are connected to a ligand, and the right wavy lines are connected to the X.

In a preferred embodiment, the linker-drug conjugate is selected from the following structures without restriction: 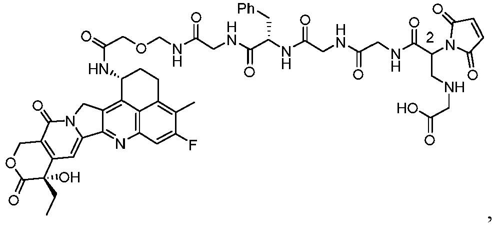 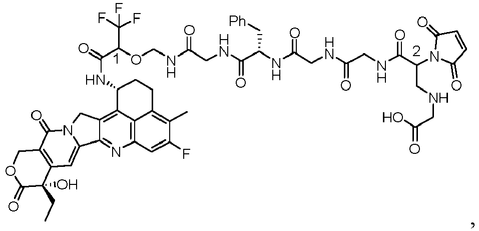 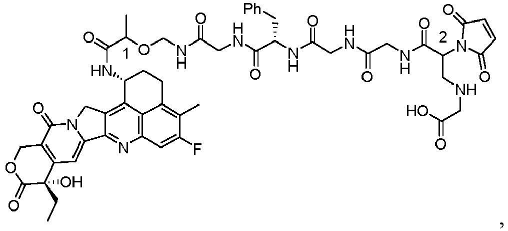 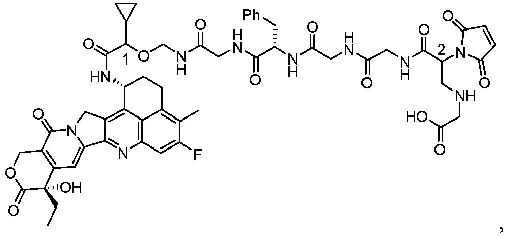 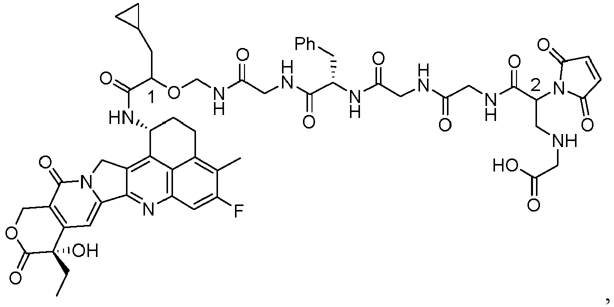 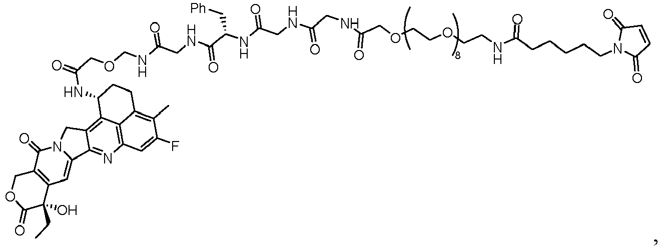 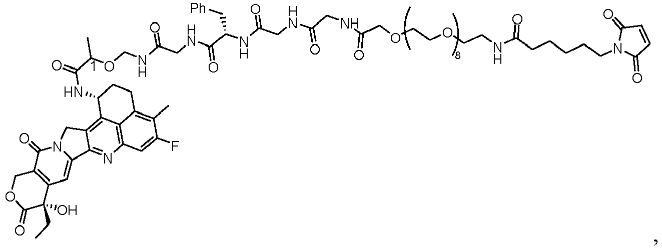 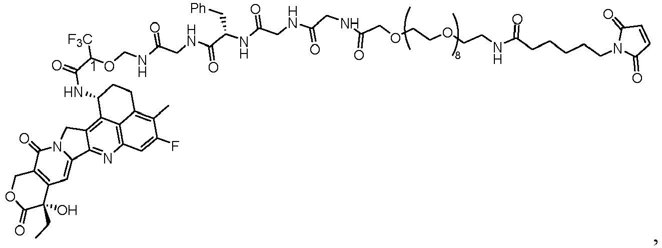 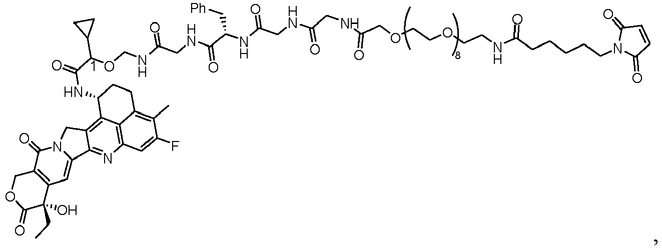 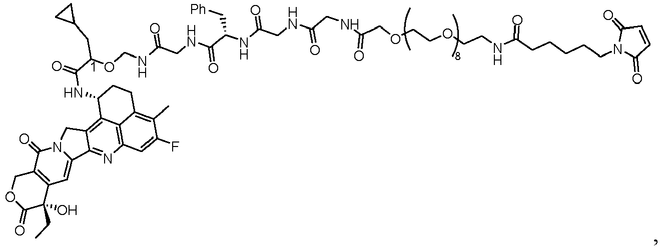 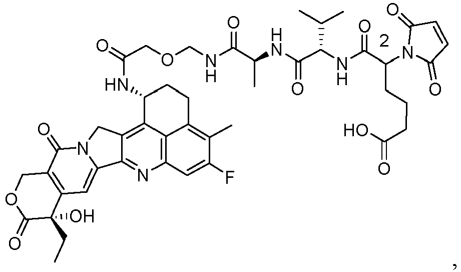 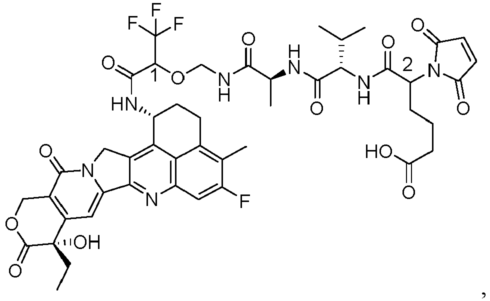 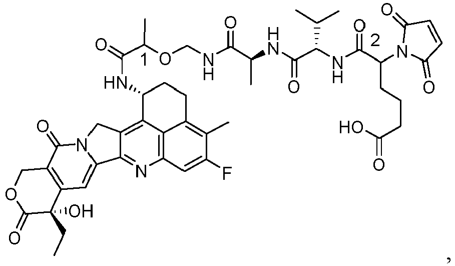 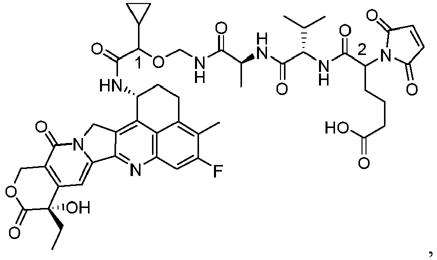 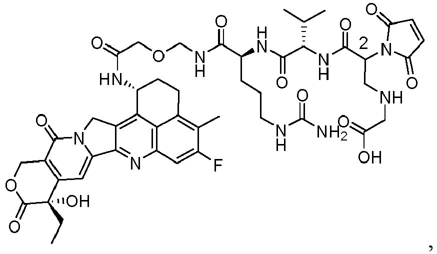 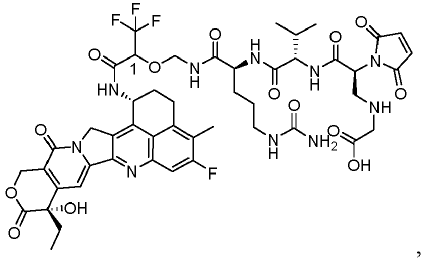 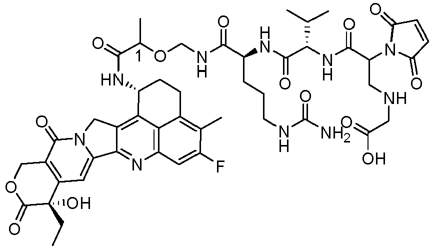 or 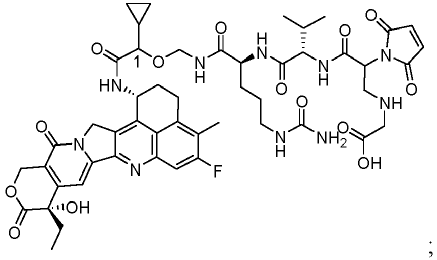 wherein:
the carbon atom at position 1 has an absolute chirality of either R or S configuration.

The instant invention also provides a ligand-drug conjugate that comprises the linker-drug conjugate, or a pharmaceutically acceptable salt or solvate thereof, wherein the ligand-drug conjugate comprises the structure of Formula I: 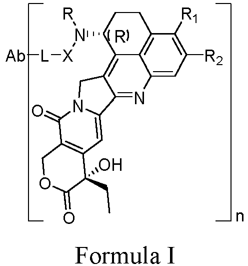 wherein:
the chiral carbon atom connected to the 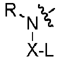 group has an absolute chirality of R configuration;
R is selected from the group consisting of hydrogen, deuterium, halogen, alkyl, substituted alkyl, deuterated alkyl, cycloalkylalkyl, alkoxy alkyl, aryl, substituted aryl and heteroaryl;
R₁ is selected from the group consisting of hydrogen, deuterium, halogen, alkyl, substituted alkyl, deuterated alkyl, cycloalkylalkyl, alkoxy alkyl, carboxyl, heterocyclic, aryl, substituted aryl and heteroaryl;
R₂ is selected from the group consisting of hydrogen, deuterium, halogen, alkyl, substituted alkyl, deuterated alkyl, cycloalkylalkyl, alkoxy alkyl, carboxyl, heterocyclic, aryl, substituted aryl and heteroaryl;
X is selected from -C(O)-CRₐR_{b}-(CR₃R₄)ₘ-O-, -C(O)-CRₐR_{b}-(CR₃R₄)ₘ-NH- or - C(O)-CRₐR_{b}-(CR₃R₄)ₘ-S-;
Rₐ is selected from the group consisting of hydrogen, deuterium, halogen, alkyl, deuterated alkyl, halogenated alkyl, cycloalkylalkyl, alkoxy alkyl, heterocyclic, aryl, substituted aryl and heteroaryl;
R_{b} is selected from the group consisting of hydrogen, deuterium, halogen, alkyl, deuterated alkyl, halogenated alkyl, cycloalkylalkyl, alkoxy alkyl, heterocyclic, aryl, substituted aryl and heteroaryl;
alternatively, Rₐ, R_{b} and their connected carbon atoms form a C₃₋₆ cycloalkyl group, a cycloalkylalkyl or a heterocyclic group;
R₃, R₄ are identical or different, and are independently selected from the group consisting of hydrogen, deuterium, halogen, alkyl, haloalkyl, deuterated alkyl, alkoxy, hydroxyl, amino, cyano, nitro, hydroxyalkyl, cycloalkyl and heterocyclic;
alternatively, R₃, R₄ and their connected carbon atoms form a C₃₋₆ cycloalkyl group, a cycloalkylalkyl or a heterocyclic group;
Ab is a ligand unit selected from antibodies, antibody fragments, targeted proteins, Fc-fusion proteins, etc.;
L is an Ab linking unit; X is a drug modification unit; and
m is selected from integers 0-4; n is selected from integers or decimals from 1-20.

In a preferred embodiment, the Ab is selected from murine antibodies, chimeric antibodies, humanized antibodies, fully human antibodies, antibody fragments, bispecific antibodies and multispecific antibodies.

In a preferred embodiment, the antibody or an antigen-binding fragment is selected without restriction from the group consisting of anti-EGFRvIII antibody, anti-DLL-3 antibody, anti-PSMA antibody, anti-CD70 antibody, anti-MLTC16 antibody, anti-ENPP3 antibody, anti-TDGF1 antibody, anti-ETBR antibody, anti-MSLN antibody, anti-TIM-1 antibody, anti-LRRC15 antibody, Anti-LIV-1 antibody, anti-CanAg/AFP antibody, anti-cladin 18.2 antibody, anti-Mesothelin antibody, anti-HER2 (ErbB2) antibody, anti-EGFR antibody, anti-c-MET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3 ( ErbB3) antibody, anti-MLTC1/CD227 antibody, anti-AXL antibody, anti-CD166 antibody, anti-B7-H3 (CD276) antibody, anti-PTK7/CCK4 antibody, anti-PRLR antibody, anti-EFNA4 antibody, anti-5T4 antibody, anti-NOTCH3 antibody, anti-Nectin 4 antibody, anti-TROP-2 antibody, anti-CD142 antibody, anti-CA6 antibody, anti-GPR20 antibody, anti-CD174 antibody, Anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody , anti-CD33 antibody, anti-CD56 antibody, anti-CD74 antibody, anti-CD22 antibody, anti-CD30 antibody, anti-CD37 antibody, anti-CD47 antibody, anti-CD138 antibody, anti-CD352 antibody, anti-CD25 antibody and anti-CD123 antibody.

In a preferred embodiment, the ligand-drug conjugate is selected from the following structures without restriction: 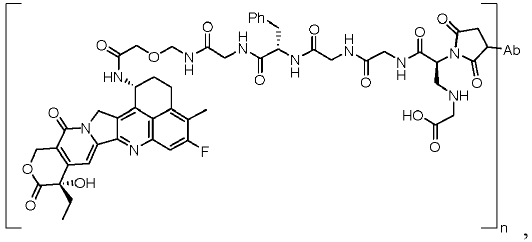 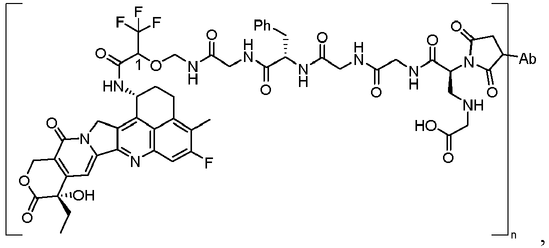 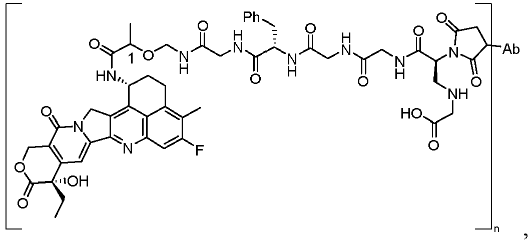 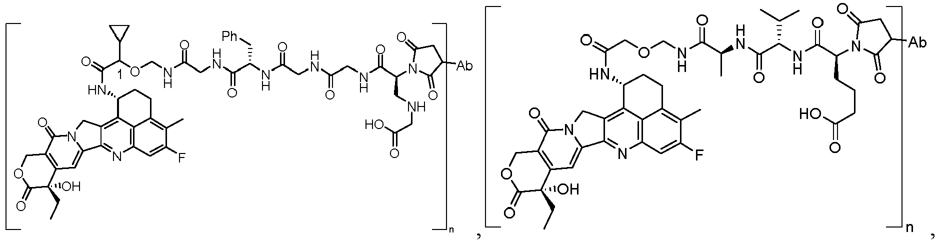 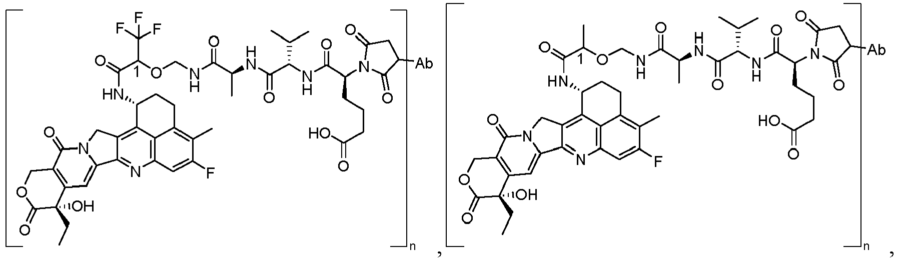 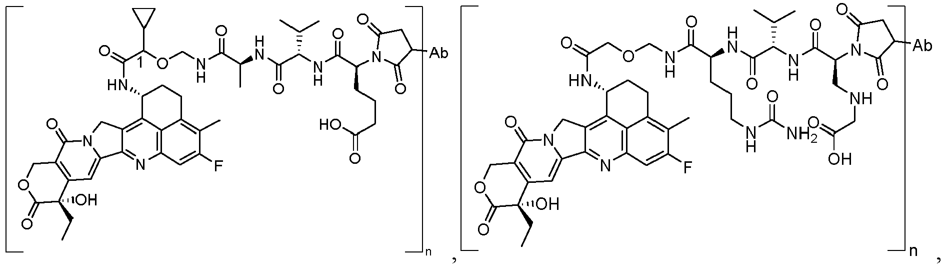 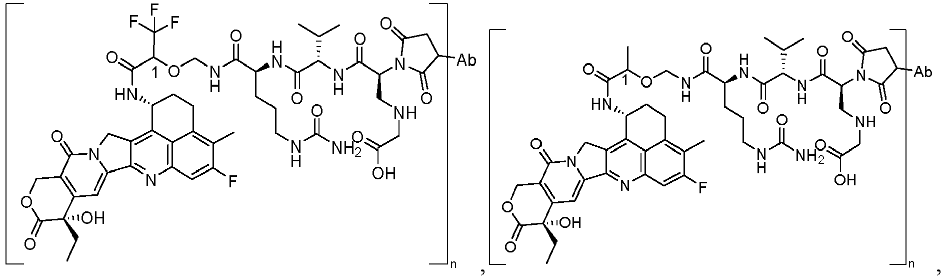 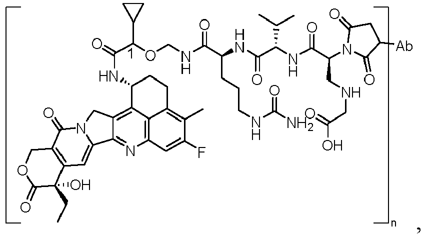 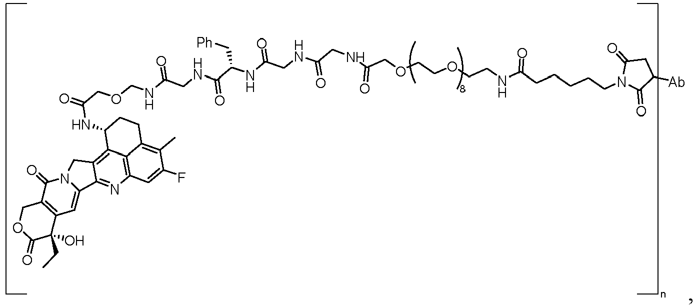 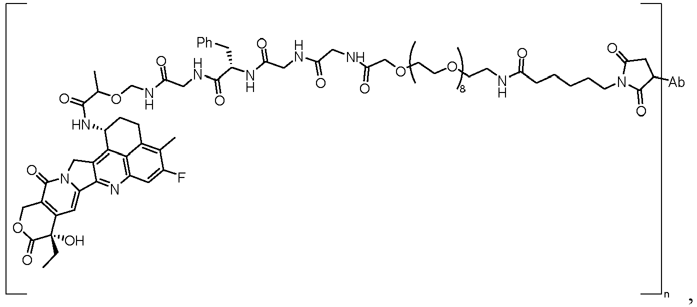 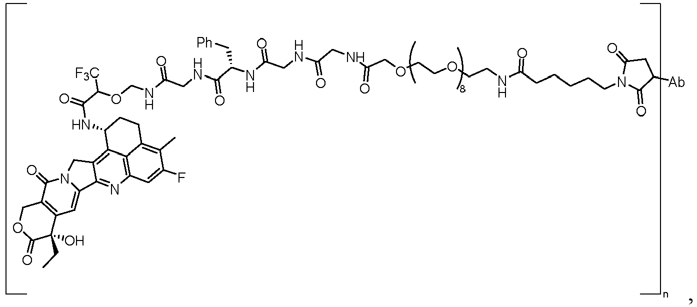 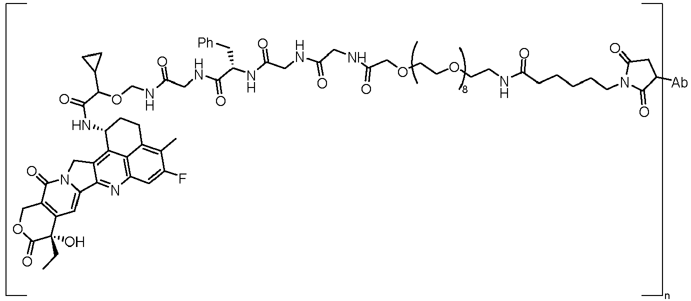 or 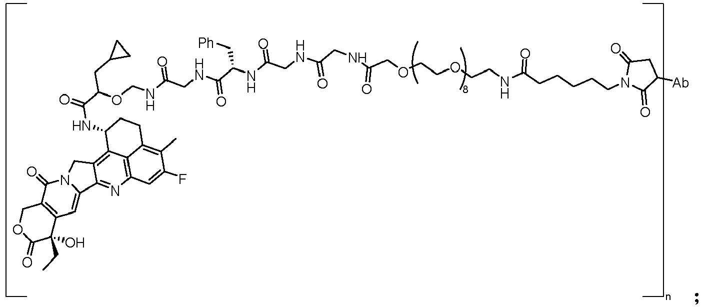 wherein:
the Ab is the ligand unit; n is selected from integers or decimals within 1-20; and
the carbon atom of the position 1 has an absolute chirality of either R or S configuration.

The instant invention also provides a method for preparing the linker-drug conjugates or their tautomers, mesomers, racemates, enantiomers, diastereoisomers or a mixture thereof, or pharmaceutically acceptable salts or solvates thereof, comprising the following steps: 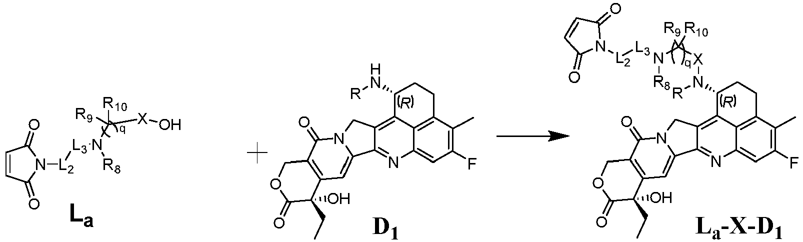
through a replacement reaction between the linking unit La and the compound of Formula D₁, obtaining the linker-drug conjugate of Formula La-X-D₁;
wherein:
   the chiral carbon atom connected to the 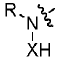 group has an absolute chirality of R configuration;
   L₂, L₃, R, R₈, R₉, R₁₀, q and X are described in Formula L-X-D.

The instant invention also provides method for preparing the ligand-drug conjugate or its tautomers, mesomers, racemates, enantiomers, diastereoisomers or a mixture thereof, or pharmaceutically acceptable salts or solvates thereof, comprising the following steps: 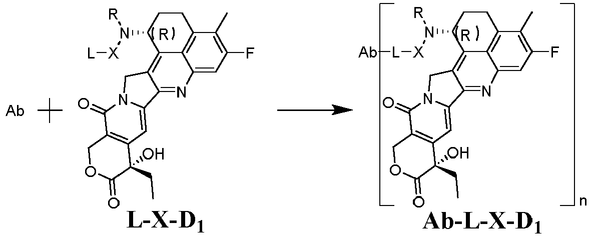
conjugating reduced antibody, antibody fragment or antigen-binding fragment with the linker-drug conjugate of Formula L-X- D₁ to obtain the ligand-drug conjugate as shown in Formula Ab-L-X-D_{1;}
wherein:
   the chiral carbon atom connected to the 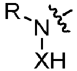 group has an absolute chirality of R configuration; and
   Ab, L, X, Rand n are described in Formula I.

The camptothecin derivative, linker-drug conjugate or ligand-drug conjugate, or a pharmaceutically acceptable salt or solvate thereof, wherein the pharmaceutically acceptable salt includes sodium, potassium, calcium or magnesium salts formed with acidic functional groups in the structural formula; or acetate, trifluoroacetate, citrate, oxalate, tartrate, malate, nitrate, chloride, bromide, iodide, sulfate, bisulfate, phosphate, lactate, oleate, ascorbate, salicylate, formate, glutamate, methanesulfonate, ethanesulfonate, benzenesulfonate or p-toluenesulfonate formed with basic functional groups in the structure.

The instant invention provides a pharmaceutical composition comprising a therapeutically effective amount of the camptothecin derivative, linker-drug conjugate or ligand-drug conjugate of claims 1-23, or a tautomer, racemate, racemate, enantiomer, enantiomer or mixture thereof, or pharmaceutically acceptable salt or solvate thereof, and a pharmaceutically acceptable carrier, diluent or excipient.

The instant invention provides the use, for purpose of preparing drugs for the treatment or prevention of tumors, of the camptothecin derivative, linker-drug conjugate or ligand-drug conjugate, or its tautomer, mesomer, racemate, enantiomer, diastereoisomer or a mixture thereof, or a pharmaceutically acceptable salt or solvent compound thereof, and a pharmaceutically acceptable carrier, diluent, or excipient.

In a preferred embodiment, the tumors comprise breast, ovarian, cervical, uterine, prostate, kidney, urethral, bladder, liver, gastric, endometrium, salivary gland cancer, esophageal cancer, lung cancer, colon cancer, rectal cancer, colorectal cancer, bone cancer, skin cancer, thyroid cancer, pancreatic cancer, melanoma, glioma, neuroblastoma, glioblastoma, sarcoma, lymphoma or leukemia.

### Detailed Description

Unless otherwise qualified, all technical and scientific terms used herein are consistent with the common understanding of those of ordinary skill in the art to which the present disclosure belongs. Although this disclosure may also be implemented or tested using any method and material similar or equivalent to the herein, the preferred method and material described herein is described herein. When describing and claiming protection of the invention, the following terms are used in accordance with the following definitions.

When a trade name is used in the present invention, the applicant intends to include the formulation of the trade name product, the generic drug and the active portion of the trade name product.

Unless stated to the contrary, terms used in the description and claims have the following meanings.

The term "ligand" refers to a macromolecular compound that recognizes and binds to antigens or receptors associated with target cells. The role of the ligands is to present drugs to target cell populations that bind to ligands, the ligands include but are not limited to protein hormones, lectins, growth factors, antibodies, or other molecules that bind to cells. In an embodiment of the present invention, the ligand is represented as Ab, and the ligand may form a bond with a linker by heteroatoms on the ligand. Preferably the ligand is an antibody or its antigen-binding fragment, the antibody is selected from a chimeric antibody, a humanized antibody, a fully human antibody or a murine antibody; preferably the Ab is monoclonal antibodies.

Ligand units refer to targeting agents that bind specifically to a target moiety. The ligand is capable of specifically binding to a cellular component or binding to a cellular component or binding to other target molecules of interest. The target portion or target is usually located on the cell surface. In some ways, the role of the ligand unit is to deliver a drug unit to the specific target cell population with which the ligand unit interacts. Ligands include, but are not limited to, proteins, polypeptides, and peptides, as well as non-proteins such as sugars. Suitable ligand units include, for example, antibodies, such as full-length (complete) antibodies or their antigen-binding fragments. In an embodiment in which the ligand unit is a non-antibody targeting reagent, it may be a peptide or polypeptide, or a non-protein molecule. Examples of such targeting agents include interferons, lymphokines, hormones, growth factors and colony-stimulating factors, vitamins, nutrient transport molecules, or any other cell-binding molecule or substance. In some embodiments, the linker is covalently linked to the sulfur atom of the ligand. In some embodiments, the sulfur atom is the sulfur atom of the cysteine residue that forms the interchain disulfide bond of the antibody. In some other embodiments, the sulfur atom is the sulfur atom of the cysteine residue that has been introduced into the ligand unit, which forms the interchain disulfide bond of the antibody. In some embodiments, the sulfur atoms are sulfur atoms that have been introduced into cysteine residues of ligand units (e.g., by site-directed mutagenesis or chemical reactions). In other respects, the linker-bound sulfur atom is selected from the cysteine residue that forms interchain disulfide bond of an antibody or the fronteceine residue that has been introduced into the ligand unit (e.g., by site-specific mutagenesis or chemical reaction). In some embodiments, EU index numbering system in Kabat (Kabat E.A. et al., (1991) (Sequences of Proteins of Immunological Interest, Fifth Edition, NIH Publication 91-3242) are used.

As used herein, "antibody" or "antibody unit" includes any part of the antibody structure to the extent to which it belongs. This unit can bind, reactively link, or form a complex to a receptor, antigen, or other receptor unit that the targeted cell population has. An antibody can be any protein or protein molecule that can bind, form a complex, or react with a portion of a cell population to be treated or biomodified. The antibody composed of an antibody drug conjugate in the present invention maintains its original antigen-binding ability in its original wild state. Therefore, the antibodies in the present invention can bind to antigens specifically. Antigens involved include, for example, tumor-associated antigens (TAAs), cell surface receptor proteins and other cell surface molecules, regulators of cell survival, regulators of cell proliferation, molecules associated with tissue growth and differentiation (such as known or predicted functional), lymphokines, cytokines, molecules involved in the regulation of the cellular cycle, molecules involved in angiogenesis, and molecules associated with angiogenesis (e.g., known or predicted functional). Tumor-associated factors can be clustered differentiating factors (e.g., CD protein).

Antibodies used in antibody-drug conjugates include, but are not limited to, antibodies against cell surface receptors and tumor-associated antigens. Such tumor-associated antigens are well known in the industry and can be prepared through well-known antibody preparation methods and information. To develop effective cell-level targets that can be used in cancer diagnosis and treatment, researchers seek transmembrane peptides or other tumor-associated peptides. These targets can be expressed specifically on the surface of one or more cancer cells and expressed little or no on the surface of one or more non-cancer cells. In general, such tumor-associated peptides tend to be overexpressed on the surface of cancer cells than on the surface of non-cancer cells. Identification of such tumor-associated factors can greatly improve the specific targeting characteristics of antibody-based treatment of cancer. For convenience, well-known antigen-related information is indicated below, including names, other names, and gene bank registry numbers. Nucleic acid and protein sequences corresponding to tumor-associated antioxidants can be found in public databases, such as Genbank. Tumor-associated antigens targeted by antibodies include all amino acid sequence variants and allogenes, have at least 70%, 80%, 85%, 90%, or 95% homology with sequences confirmed in the references, or have biological properties and characteristics that are completely consistent with the tumor-associated antigen sequences in the cited literature.

The term "inhibit" or "inhibition" refers to reducing the amount that can be detected, or preventing it altogether.

The term "cancer" refers to a physiological condition or disease characterized by dysregulated cell growth. "Tumor" includes cancer cells.

The term "autoimmune disease" is a disease or disorder that originates in targeting an individual's own tissues or proteins.

The term "drug" refers to a cytotoxic drug, expressed as D, a chemical molecule that can disrupt tumor cell normal growth when present within the tumor cell. In principle, cytotoxic drugs can kill tumor cells at high enough concentrations, but due to the lack of specificity, while killing tumor cells, a cytotoxic drug may also lead to apoptosis of normal cells, resulting in serious side effects. The term includes toxins such as small molecule toxins or enzyme-active toxins of bacterial, fungal, plant or animal origin, radioisotopes (e.g., At²¹¹,I¹³¹ ,I¹²⁵,Y⁹⁰,Re¹⁸⁶,Re¹⁸⁸, Sm¹⁵³,Bi²¹²,P³² and Lu), toxic drugs, chemotherapy drugs, antibiotics and ribozin, preferably toxic drugs.

The term "linker" or "linker fragment" or "linker unit" refers to a chemical structure fragment or bond that is attached/connected/lined to a ligand at one end and to a drug at the other end, and can also be attached/connected/lined to other connectors and then connected to a drug.

Connectors, including extensions, spacers and amino acid units, can be synthesized by methods known in the art, such as those described in US2005-0238649A1. Adapters can be "cuttable junctions" that facilitate the release of drugs in the cell. For example, acid-labile (e.g., hydrazone), protease-sensitive (e.g., peptidase-sensitive) connector, photolabile connector, dimethyl connector, or disulfide-containing connector may be used (Chari et al. Cancer Research 52: 127-131, 1992); U.S. Patent No. 5, 208, 020.

Depending on the mechanism of drug release in the cell, as used herein, "linkers" or "linkers of antibody-drug conjugates" can be divided into two categories: non-breakable linkers and breakable linkers. For antibody drug conjugates containing non-breakable linkers, the drug release mechanism is: after the conjugates bind to antigens and are endocytosed by cells, the antibodies are enzymatically hydrolyzed in lysosomes, releasing active molecules composed of small molecule drugs, linkers, and antibody amino acid residues. The resulting changes in the molecular structure of the drug do not diminish its cytotoxicity, but because the active molecule is charged (amino acid residues), it cannot penetrate into neighboring cells. Therefore, such active drugs cannot kill tumor cells (bystander effect) that do not express a targeted antigen (antigen-negative cells) (Ducry et al., 2010, Bioconjugate Chem. 21: 5-13).

The term "ligand-drug conjugate" refers to the antibody being attached to a biologically active drug through a stable linker. In the present invention, "ligand-drug conjugate" is preferably an antibody drug conjugate (ADC), which refers to a monoclonal antibody or antibody fragment connected to a biologically active toxic drug through a stable linker unit.

The amino acid three-letter code and the single-letter code used in the present disclosure are such as J. boil. Chem. 1968, 243, 3558.

The term "alkyl" refers to a saturated aliphatic hydrocarbon group, which include a straight-chain or branched-chain group comprising 1 to 20 carbon atoms, preferably an alkyl group comprising 1 to 12 carbon atoms, more preferably an alkyl group containing 1 to 10 carbon atoms, more preferably an alkyl group containing 1 to 6 carbon atoms. Non-limiting examples include methyl, ethyl, n-propyl, isopropyl, n-butyl, isobutyl, tert-butyl, sec-butyl, n-pentyl, 1, 1-dimethylpropyl, 1, 2-dimethylpropyl, 2, 2-dimethylpropyl, 1-ethylpropyl, 2-methylbutyl, 3-methylbutyl, n-hexyl, 1-ethyl-2-methylpropyl, 1, 1, 2-trimethylpropyl, 1, 1-dimethylbutyl, 1, 2-dimethylbutyl, 2, 2-dimethylbutyl, 1, 3-dimethylbutyl, 2- Ethyl butyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl,2,2-dimethylpentyl,3,3-dimethylpentyl,2-ethylpentyl,3-ethylpentyl,3-ethylpentyl,n-octyl,2,3-dimethylhexyl,2,4-dimethylhexyl,2,5-dimethylhexyl,2, 2-Dimethylhexyl, 3, 3-dimethylhexyl, 4, 4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2-diethylpentyl, n-decyl, 3, 3-diethylhexyl, 2, 2-diethylhexyl, and various branched-chain isomers. More preferably, a lower alkyl group comprising 1 to 6 carbon atoms, non-limiting embodiments comprising methyl, ethyl, n-propyl, isopropyl, n-butyl, isobutyl, tert-butyl, sec-butyl, n-pentyl, 1, 1-dimethylpropyl, 1, 2-dimethylpropyl, 2-dimethylpropyl, 1-ethylpropyl, 2-methylbutyl, 3-methylbutyl, n-hexyl, 1-ethyl-2-methylpropyl, 1, 1, 2-trimethylpropyl, 1, 1-dimethylbutyl, 1, 2-dimethylbutyl, 2, 2- Dimethylbutyl, 1,3-dimethylbutyl,2-ethylbutyl,2-methylpentyl,3-methylpentyl,4-methylpentyl group,2,3-dimethylbutyl,etc. Alkyl groups may be substituted or non-substituted, when substituted, the substituent may be substituted at any usable junction, the substituent is preferably one or more of the following groups, which are independently selected from alkyl groups, alkenyl groups, alkynyls, alkoxy, alkyl aminos, halogens, mercapto, hydroxyl, nitro, cyano, cycloalkyl, heteronyl, aryl, heteroaryl, cycloalkoxy, heteronic alkoxy, cycloalkylthio, heteronic thio, and oxo.

The term "substituted alkyl" refers to that hydrogen in an alkyl group is substituted by a substituent group, and unless otherwise stated in the text, the substituents of an alkyl group may be a variety of groups selected from the following group: -OR',-NR'R",-SR',-SiR'R"R‴,-OC(O)R',-C(O)R',-CO₂R',-CONR'R",-OC(O)NR'R",-NR"C ( O ) R',-NR'-C(O)NR"R‴,-NR"C(O)₂R',-NH-C(NH₂)=NH,-NR'C(NH₂)=NH,-NH-C(NH₂)=NR',-S(O)R',-S(O)₂R',-S(O)₂NR'R",-NR'S(O)₂R",-CN and -NO₂, with the number of substituents from 0 to (2m'+1), where m' is the total number of carbon atoms in the group. R',R"and R‴ each independently refer to hydrogen, unsubstituted C₁₋₈ alkyl, unsubstituted aryl, aryl group substituted by 1-3 halogens, unsubstituted C₁₋₈ alkyl, C₁₋₈ alkoxy or C₁₋₈ thioalkoxy, or unsubstituted aryl-C₁₋₄ alkyl. When R' and R" are attached to the same nitrogen atom, they can form a 3-, 4-, 5-, 6- or 7- ring with that nitrogen atom. For example, -NR'R" includes 1-pyrrolidinyl and 4-morpholinoyl.

The term "heteralkyl" refers to an alkyl group containing one or more heteroatoms selected from N, O or S, wherein the alkyl group is defined above.

The term "alkylene" refers to a saturated linear or branched aliphatic hydrocarbon group, which has 2 residues derived from the same carbon atom or two different carbon atoms of the parent alkane removed from two hydrogen atoms, which is a straight chain or branched chain group comprising 1 to 20 carbon atoms, preferably containing 1 to 12 carbon atoms, preferably an alkylene group containing 1 to 6 carbon atoms. Non-limiting examples of alkyl include, but are not limited to, methylene (-CHz-,1, 1-ethylene (-CH(CH₃)-), 1, 2-ethylene (-CH₂CH₂)-, 1, 1-propylidene(-CH(CH₂CH₃)-), 1, 2-propylidene(-CH2CH(CH₃)-), 1, 3-propylidene(-CH₂CH₂CH₂-), 1, 4-butylidene(-CH₂CH₂CH₂CH₂-) and 1, 5-butylidene(- CH₂ CH₂CH₂CH₂CH₂-) and so on. Alkylene may be substituted or non-substituted, when substituted, the substituent may be substituted at any usable junction, the substituent preferably independently selected from one or more substituents in alkyl, alkenyl, alkyny, alkoxy, alkylamino, halogen, mercapto, hydroxyl, nitro, cyano, cycloalkyl, heterocyclic group, aryl, heteroarylaryl, cycloalkoxy, heterocycloalkoxy, heterocyclothio, Cycloalkylthio thio, heteronic sulfide and oxo.

The term "alkoxy" refers to -O- (alkyl) and -O- (cycloalkyl), where alkyl or cycloalkyl is defined as described above. Non-limiting examples of alkoxy groups include: methoxy, ethoxy, propoxy, butoxy, cyclopropoxy, cyclobutoxy, cyclopentoxy, cyclohexoxy. Alkoxy groups may be optionally substituted or non-substituted, when substituted, the substituents are preferably one or more of the following groups, which are independently selected from alkyl groups, alkenyl groups, alkynyls, alkyl aminos, halogens, mercapto, hydroxyl, nitro, cyano, cycloalkyl, heteronyl, aryl, heteroaryl, naphthenoxy, heteronic alkoxy, cycloalkylthio, heteronic thio, and heteronic sulfonyl.

The term "cycloalkyl" refers to saturated or partially unsaturated monocyclic or polycyclic hydrocarbon substituents, the cycloalkyl ring comprises 3 to 20 carbon atoms, preferably comprising 3 to 12 carbon atoms, more preferably comprising 3 to 10 carbon atoms, and most preferably comprising 3 to 8 carbon atoms. Non-limiting examples of monocyclocycloalkyl include cyclopropyl, cyclobutyl, cyclopentyl, cyclopentenyl, cyclohexenyl, cyclohexenyl, cyclohexadienyl, cycloheptyl, cycloheptyl, cycloheptyl, cycloheptyl, cyclooctyl, etc.; Polycyclic s groups include cycloalkyl groups of spirocyclical, polycyclic and bridge rings.

The term "heterocyclic group" refers to a saturated or partially unsaturated monocyclic or polycyclic hydrocarbon substituent containing 3 to 20 ring atoms, of which one or more ring atoms are heteroatoms selected from nitrogen, oxygen, or S(O)m (where m is an integer from 0 to 2), but excluding the ring portion of -O-O-, -O-S- or -S-S-, and the remaining ring atoms are carbon. Preferably containing 3 to 12 ring atoms, of which 1~4 are heteroatoms; More preferably, the cycloalkyl ring comprises 3 to 10 ring atoms. Non-limiting examples of monocyclic heterocyclics include pyrrolidinyl, piperidinyl, piperazinyl, morpholinoyl, thiomorpholinoline, homopiperazinyl and the like. Polycyclic heterocyclic groups include heterocyclic groups of spirocyclic rings, poly rings, and bridge rings.

The term "spiroheterocyclic group" refers to a polycyclic heterocyclic group that shares an atom (called a spiro atom) between a single ring of 5 to 20 ring atoms, where one or more ring atoms are heteroatoms selected from nitrogen, oxygen or S (O) m (where m is integers 0 to 2), and the remaining ring atoms are carbon. It may contain one or more double bonds, but none of the rings have a fully conjugated vulon system. Preferably 6 to 14 ring atoms, more preferably 7 to 10 ring atoms. According to the number of spiral atoms shared between rings, spiral heterocyclic groups can be divided into single, double or multiple spiral heterocyclic groups, preferably single-spiral heterocyclic group and double-spiral heterocyclic group. More preferably 4 ring atoms/4 ring atoms, 4 ring atoms/5 ring atoms, 4 ring atoms/6 ring atoms, 5 ring atoms/5 ring atoms or 5 ring atoms/6 ring atoms single-spiral heterocyclic group.

The term "cycloalkylalkyl" means that an alkyl group is replaced by one or more cycloalkyl groups, preferably by one, where an alkyl group is defined above, and where a cycloalkyl group is defined above.

The term "alkyl halide" refers to the substitution of alkyl by one or more halogens, of which alkyl is defined above.

The term "deuteryl alkyl" refers to the substitution of an alkyl group with one or more deuterium atoms, with alkyl groups as defined above.

The term "hydroxyl" refers to the -OH group.

The term "halogen" refers to fluorine, chlorine, bromine, or iodine.

The term amino refers to NH₂. The term "nitro" refers to -NO₂.

The term "amide group" means -C(O)N(alkyl) or (cycloalkyl), where alkyl, cycloalkyl are defined above.

The term "carboxylate group" means -C(O)O(alkyl) or (cycloalkyl), where alkyl, cycloalkyl are defined above.

The invention also includes various forms of deuteration. Each available hydrogen atom attached to a carbon atom can be independently replaced by a deuterium atom. A person skilled in the art of this field can synthesize deuterated compounds by referring to relevant literature. In the preparation of deuterium compounds, commercially available deuterium initiation materials may be used, or they may be synthesized using conventional techniques using deuterium reagents. Unrestricted examples of deuterium reagents include: deuterium methanol, deuterium water, deuterium borane, trideuterium borane tetrahydrofuran solution, deuterium aluminum hydride, deuterium iodide ethane and deuterium iodide methane.

The term "antibody" refers to immunoglobulin, a tetrapeptide structure consisting of two identical heavy chains and two identical light chains connected by interchain disulfide bonds. The amino acid composition and sequence of the heavy chain constant region of immunoglobulin are different, so its antigenicity is also different. On this basis, immunoglobulins can be divided into five classes, or isotypes of immunoglobulins, namely IgM, IgD, IgG, IgA, and IgE, with their corresponding heavy chains being µ, δ, γ, α, and ε chain. Ig can be divided into different subgroups according to the amino acid composition of hinge region and the number and location of heavy chain disulfide bonds. For example, IgG can be divided into IgG1, IgG2, IgG3, and IgG4. The light chain is divided into κ chain or λ chain through the difference of the constant region. Each of the five types of Ig can have a κ chain or a λ chain. The antibody of the invention is preferably a specific antibody against the cell surface antigen on the target cell, and the non-restrictive embodiments are the following: Anti-egfrviii antibody, anti-DLL-3 antibody, anti-Psma antibody, anti-CD70 antibody, anti-MUC16 antibody, anti-ENPP3 antibody, anti-tDGF1 antibody, anti-ETbr antibody, anti-MSLn antibody, anti-Tim-1 antibody, anti-LrRC15 antibody, anti-Liv-1 antibody, anti-Canag /AFP antibody , anti-cladin 18.2 Antibody, anti-Mesothelin antibody, anti-HER2 (ErbB2) antibody, anti-EGFR antibody, anti-C-Met antibody, anti-SlitrK6 antibody, anti-Kit /CD117 antibody, anti-Steap1 antibody, Anti-SlamF7 /CS1 antibody, anti-Napi2b /SLC34A2 antibody, Anti-GPNMB antibody, anti-HER3 (ErbB3) antibody, anti-MLTC1 /CD227 antibody, anti-Axl antibody, anti-CD166 antibody, anti-B7-H3 (CD276) antibody, anti-PTK7 /CCK4 antibody, anti-PRLR antibody, anti-EFna4 antibody, anti-5T4 antibody, anti-NotCH3 antibody, anti-NEC antibody tin 4 Antibody, anti-Trop-2 antibody, anti-CD142 antibody, anti-CA6 antibody, anti-GPR20 antibody, anti-CD174 antibody, anti-CD71 antibody, anti-Epha2 antibody, anti-LypD3 antibody, anti-FGFR2 antibody, anti-FGFR3 antibody, anti-Frα antibody, anti-Ceacams antibody, anti-GCC antibody, anti-IN antibody tegrin Av antibody, anti-Caix antibody, anti-P-Cadherin antibody, anti-GD3 antibody, anti-Cadherin antibody 6 Antibody, anti-LAMp1 antibody, anti-flT3 antibody, anti-BCMA antibody, anti-CD79B antibody, anti-CD19 antibody, anti-CD33 antibody, anti-CD56 antibody, anti-CD74 antibody, anti-CD22 antibody, anti-CD30 antibody, anti-CD37 antibody, anti-CD138 antibody, anti-CD352 antibody, anti-CD25 antibody or one or more anti-CD123 antibodies; preferably Trastuzumab (trade name Herceptin), Pertuzumab (also known as 2C4, trade name Perjeta), Nimotuzumab (Nimotuzumab, Trade name tai xin), Enoblituzumab Emibetuzumab, Inotuzumab, Pinatuzumab Brentuximab, Gemtuzumab Bivatuzumab, Lorvotuzumab cBR96 and Glematumamab.

The term "solvate" or "solvent compound" means that the ligand-drug conjugate of the present invention forms a medically-usable solvate with one or more solvent molecules, non-restrictive examples of which include water, ethanol, acetonitrile, isopropyl alcohol, DMSO, ethyl acetate.

The term "drug load" refers to the average number of cytotoxic drugs loaded on each antibody in the Formula I molecule, and can also be expressed as the ratio of drug dose to antibody dose. The drug load can range from 0 to 12 for each antibody (Ab), preferably 1 to 10 cytotoxic drugs (D). In the embodiments of the invention, the drug load is expressed as n, which, exemplary, can be the mean of 1, 2, 3, 4, 5, 6, 7, 8, 9, 10. Conventional methods such as UV/visible light spectroscopy, mass spectrometry, ELISA assay, and HPLC characterization were used to identify the average amount of drug for each ADC molecule after coupling reaction.

In one embodiment of the present invention, a cytotoxic drug is coupled to the n-terminal amino group of a ligand and/or the ε-amino group of a lysine residue by a linking unit. In general, the number of drug molecules that can be coupled to an antibody in the coupling reaction will be less than the theoretical maximum.

The following non-limiting methods can be used to control the load of ligand cytotoxic drug conjugates, including:
(1) Control the molar ratio of linking reagent and monoclonal antibody,
(2) Control reaction time and temperature,
(3) Select different reaction reagents.

See Chinese Pharmacopoeia for the preparation of conventional pharmaceutical compositions.

The term " pharmaceutically acceptable salt" or "medicinal salt" means the salt of the ligand-drug conjugate of the invention, or the salt of a compound described in the invention, which is safe and effective when used in mammals and has the expected biological activity. The ligand-drug conjugate of the invention contains at least one carboxyl group and can therefore form a salt with a base. Non-restrictive examples of pharmaceutically acceptable salts include: sodium salts, potassium salts, calcium salts or magnesium salts.

The term "pharmaceutically acceptable salt" or "medicinal salt" refers to a salt of a ligand-drug conjugate of the present invention, or a salt of a compound described in the present invention, which is safe and effective when used in mammals and has the expected biological activity. The ligand-drug conjugate compound of the present invention contains at least one amino group and can therefore form salts with acids. Non-restrictive examples of pharmaceutically acceptable salts include: hydrochloride, hydrobromide, hydroiodate, sulfate, bisulfate, citrate, acetate, succinate, ascorbate, oxalate, nitrate, pearate, hydrogen phosphate, dihydrogen phosphate, salicylate, hydrogen citrate, tartrate, maleate, fumarate, formate, benzoate, methanesulfonate, ethanesulfonate, benzenesulfonate, and p-toluenesulfonate.

"Acidic amino acid" means that the isoelectric point of amino acid is less than 7, and acidic amino acid molecules often have one or more acidic groups such as carboxyl groups, which can effectively ionize into negative ion form and increase hydrophilicity in the structure. Acidic amino acids can be natural or unnatural.

"Natural amino acids" refer to amino acids that are biosynthesized. Natural amino acids are generally L-type, but there are a few exceptions, such as glycine, both natural and biosynthetic.

"Unnatural amino acids" refer to amino acids obtained by synthetic means.

The present invention is further elaborated below in conjunction with specific embodiments, it should be understood that these embodiments are only used to illustrate the present invention, and are not intended to limit the scope of the present invention. The following embodiments do not indicate specific conditions of the test method, usually in accordance with conventional conditions or in accordance with the conditions recommended by the manufacturer. Unless otherwise stated, all percentages, proportions, ratios, or servings are by weight.

Unless otherwise defined, all professions and sciences used herein are used in the same sense as those familiar to those skilled in the art.

Further, any method and material similar or equal to the content described may be applied to the method of the present invention. The best practices and materials described herein are for demonstration purposes only.

### Example 1: Synthesis of Compound 1 and iso-1

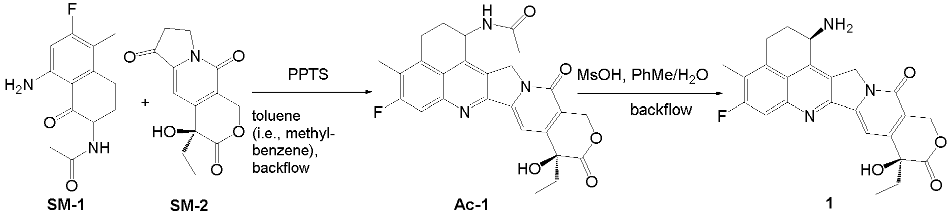

To 1L round bottom flask weigh in Compound **SM-1** (N-(8-amino-6-fluoro-5-methyl-1-oxo-1,2,3,4-tetrahydronaphthal-2-yl-acetamide, purchased) (6.25g, 25.0mmol), Compound **SM-2** ((S)-4-ethyl-4-hydroxy-7,8-dihydro-1H-pyran O[3,4-F]indolazine-3,6,10(4H)-ketone, purchased) (7.25g, 27.5mmol), Pyridine p-toluenesulfonate salt (1.26g, 5.0mmol), add 500mL of toluene, heat reflux water distribution, react for 5 hours, HPLC monitoring reaction until SM-2 is less than 10%, stop the reaction. After cooling and decompression, the solution was concentrated to half of its volume. Then the solution was stirred and crystallized in an ice bath for 2h, filtered, washed three times in filter cake with 30mL toluene, and vacuum dried to obtain compound Ac-1: 10.68g, 89%. [M+H]⁺: 478.2 is obtained by LC-MS.

**Ac-1** (10.68g, 22.5mmol), mesylate (55mL), water (110mL) and toluene (55mL) were mixed in a 1L round-bottomed flask and reflow was heated. After 4 hours, TLC monitoring the reaction, the raw material disappeared, and the reaction system was cooled to room temperature. At room temperature, 500mL methanol was slowly added into the system and continuously stirred. After the drip was completed, the reaction system was stirred overnight. Then, the reaction system was directly filtered, the filter cake was washed with methanol, the filtrate was collected, and the filter cake was dried to obtain an off-white solid compound, which was the isomer of compound 1 exatecan. Light brown solid (5.07g, 41%) was obtained by liquid phase preparation and purification. [M+H]⁺: 436.2 was obtained by LC-MS.

### Example 2: Synthesis of Compound 2

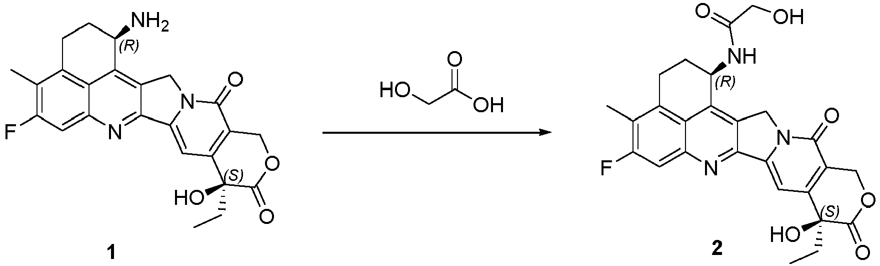

In 25ml round-bottomed flask add Compound 1 (in the form of trifluoroacetic acid) (100.0mg, 0.188mmol), glycolic acid (43.0mg, 0.565mmol), HATU (107.2mg, 0.282mmol), HOBt(38.1mg, 0.282mmol) and 5ml ultra-dry DMF, liquid sealing at the mouth of the flask. Stir the flask under a bath of ice water for 10 minutes, then add DIEA (94uL, 0.565mmol). TLC was used to monitor the reaction progress, react for anout 3h. After the reaction was complete, the reaction liquid was sent for preparation and separation. The preparation liquid was concentrated under vacuum at 35°C and lyophilized to obtain white solid Compound 2: 63.8 mg with yield of 69.7%. LC-MS results show [M+H]⁺: 493.9_{∘} ¹H NMR (400 MHz, DMSO-d6) δ 8.39 (d, J = 9.0 Hz, 1H), 7.68 (d, J = 10.9 Hz, 1H), 7.29 (s, 1H), 6.51 (s, 1H), 5.53 (dt, J = 9.1, 6.0 Hz, 1H), 5.41 (s, 2H), 5.12 (d, J = 19.1 Hz, 1H), 4.92 (d, J = 18.9 Hz, 1H), 3.99 (s, 2H), 3.10 (qt, J = 16.7, 6.1 Hz, 2H), 2.29 (d, J = 1.8 Hz, 3H), 2.16 (q, J = 6.4 Hz, 2H), 2.00 - 1.81 (m, 2H), 0.91 (t, J = 7.3 Hz, 3H).

### Example 3: Synthesis of Compound 3

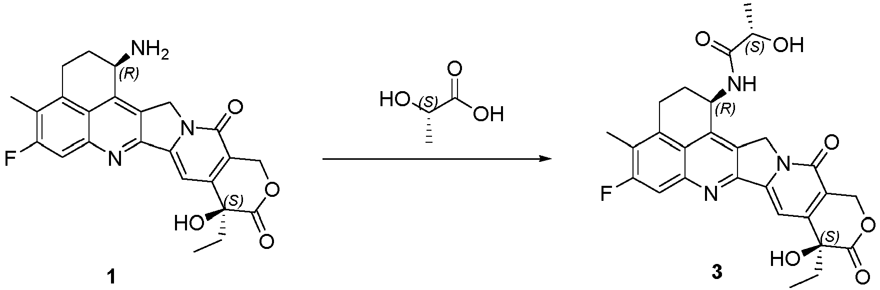

In 25ml round-bottomed flask add Compound 1 (in the form of trifluoroacetate) (100.0mg, 0.188mmol), L-lactic acid (56.6mg, 0.565mmol), HATU (107.2mg, 0.282mmol), HOBt (38.1mg, 0.282mmol) and 5ml ultra-dry DMF, liquid seal at the mouth of the flask. Stir the flask under a bath of ice water for 10 minutes, then add DIEA (94uL, 0.565mmol). The reaction progress was monitored by HPLC, and the reaction lasted about 3 hours. After the reaction was complete, saturated ammonium chloride aqueous solution was added into the reaction solution, and the light-yellow crude product was extracted and filtered. The crude product was separated by TLC preparation plate scraping plate. 42.0mg of light-yellow solid Compound **3** was obtained with yield of 46.1%.

¹H NMR (400 MHz, DMSO-d6) δ 8.46 (d, J = 9.2 Hz, 1H), 7.71 (d, J = 10.9 Hz, 1H), 7.29 (s, 1H), 6.53 (s, 1H), 5.68 (d, J = 4.9 Hz, 1H), 5.54 (q, J = 7.3 Hz, 1H), 5.42 (s, 2H), 5.20 (d, J = 19.0 Hz, 1H), 4.93 (d, J = 18.9 Hz, 1H), 4.20 - 4.08 (m, 1H), 3.25 - 3.01 (m, 2H), 2.32 (s, 3H), 2.14 (q, J = 6.5 Hz, 2H), 1.96 - 1.80 (m, J = 7.1 Hz, 2H), 1.42 (d, J = 6.8 Hz, 3H), 0.90 (t, J = 7.2 Hz, 3H).

### Example 4: Synthesis of Compound 4

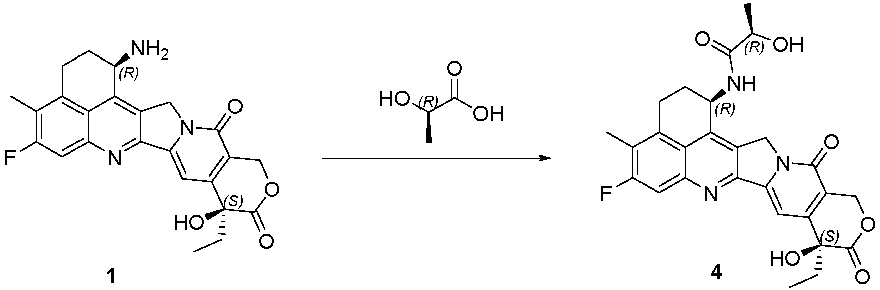

In 25ml round-bottomed flask add Compound 1 (in the form of trifluoroacetate) (100.0mg, 0.188mmol), D-lactic acid (56.6mg, 0.565mmol), HATU (107.2mg, 0.282mmol), HOBt (38.1mg, 0.282mmol) and 5ml ultra-dry DMF, liquid seal at the mouth of the flask. Stir the flask under a bath of ice water for 10 minutes, then add DIEA (94uL, 0.565mmol). The reaction progress was monitored by HPLC, and the reaction lasted about 3 hours. After the reaction was complete, the saturated ammonium chloride aqueous solution was added into the reaction solution, and a light-yellow crude product was extracted and filtered. The crude product was separated by TLC preparation plate scraping plate, and the solid was purified by beating with methylene chloride and methanol. 16.8mg of light-yellow solid Compound 4 was obtained, and the yield was 18.4%.

LC-MS results show [M+H]⁺: 508.2_{∘}

### Example 5: Synthesis of Compound 5 and 6

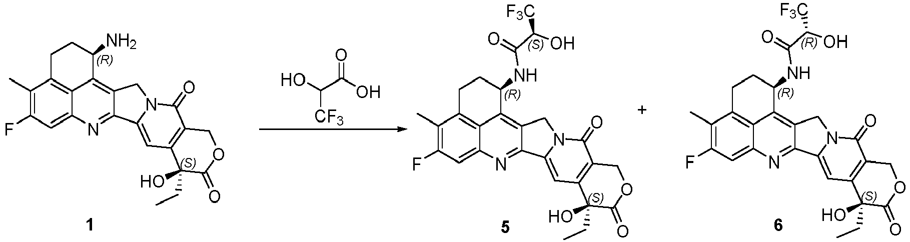

In 25ml round-bottomed flask add Compound 1 (in the form of trifluoroacetate) (200mg, 0.377mmol), trifluorolactic acid (162.8mg, 1.13mmol), HATU (214.8mg, 0.565mmol), HOBt(76.3mg, 0.565mmol) and 5ml ultra dry DMF with liquid seal at the mouth of the bottle. Stir the flask under an ice water bath for 10 minutes, then add DIEA (187uL, 1.13mmol). The reaction progress was monitored by HPLC, and the reaction lasted for about 6h. After the reaction was complete, the liquid phase was prepared, separated and purified, and the prepared liquid was concentrated at 35°C under vacuum and lyophilized to obtain 49.7mg of light-yellow solid compound **5** with yield of 48.7%. The yield of 60.5mg light yellow solid Compound **6** was 59.3%

### Compound 5:

LC-MS results show [M+H]⁺: 562.2.

¹H NMR (400 MHz, DMSO-d6) δ 9.00 (d, J = 8.7 Hz, 1H), 7.78 (d, J = 10.9 Hz, 1H), 7.31 (s, 1H), 7.19 (d, J = 6.9 Hz, 1H), 6.54 (s, 1H), 5.59 (dt, J = 10.1, 5.3 Hz, 1H), 5.43 (s, 2H), 5.16 (q, J = 19.2 Hz, 2H), 4.63 (p, J = 7.6 Hz, 1H), 3.14 (s, 2H), 2.38 (s, 3H), 2.16 (p, J = 7.1 Hz, 2H), 1.87 (hept, J = 7.1 Hz, 2H), 0.88 (t, J = 7.3 Hz, 3H).

### Compound 6:

LC-MS results show [M+H]⁺: 562.2.

¹H NMR (400 MHz, DMSO-d6) δ 8.97 (d, J = 8.6 Hz, 1H), 7.78 (d, J = 11.0 Hz, 1H), 7.31 (s, 1H), 7.26 (d, J = 6.7 Hz, 1H), 6.54 (s, 1H), 5.57 (dt, J = 9.5, 5.3 Hz, 1H), 5.43 (s, 2H), 5.24 - 5.03 (m, 2H), 4.63 (p, J = 7.4 Hz, 1H), 3.14 (t, J = 6.5 Hz, 2H), 2.38 (d, J = 1.9 Hz, 3H), 2.28 - 2.07 (m, 2H), 1.95 - 1.78 (m, J = 7.1 Hz, 2H), 0.88 (t, J = 7.3 Hz, 3H).

### Example 6: Synthesis of Compound 7 and 8

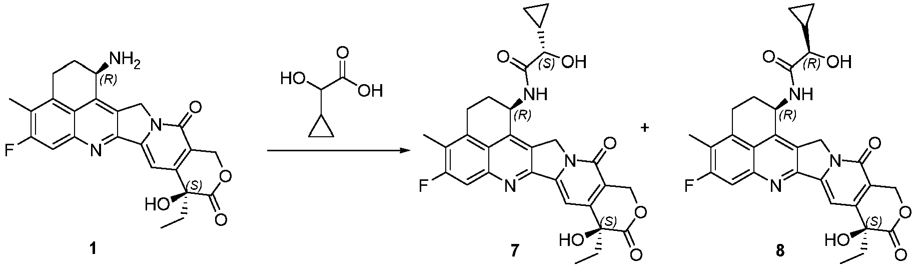

In 25ml round-bottomed flask add Compound 1 (in the form of trifluoroacetate) (200mg, 0.377mmol), 1-hydroxycyclopropane carboxylic acid (131.2mg, 1.13mmol), HATU (214.8mg, 0.565mmol), HOBt(76.3mg, 1.13mmol) and 5ml ultra dry DMF. Liquid seal at the mouth of the bottle. Stir the flask under an ice water bath for 10 minutes, then add DIEA (187uL, 1.13mmol). The reaction progress was monitored by HPLC, and the reaction lasted for about 6h. After the reaction is complete, the solid is precipitated by adding water to the reaction liquid. The saturated ammonium chloride aqueous solution is washed twice and the pure water solution is washed once. After draining, the solid is dissolved by methylene chloride and methanol. TLC preparation plate scraping plate separation. 81.0mg light-yellow solid Compound **7** was obtained with yield of 82.4%. The yield of 60.0 light yellow solid Compound **8** was 61.8%

### Compound 7: LC-MS results show [M+H]⁺: 534.2.

¹H NMR (400 MHz, d6DMSO) δ 8.40 (d, J = 9.0 Hz, 1H), 7.78 - 7.62 (m, 1H), 7.29 (s, 1H), 6.52 (s, 1H), 5.58 (dt, J = 5.3, 1.4 Hz, 1H), 5.53 (q, J = 7.5 Hz, 1H ), 5.42 (s, 2H), 5.26 - 4.91 (m, 2H), 3.62 (t, J = 5.8 Hz, 1H), 3.23 - 3.02 (m, 2H), 2.30 (d, J = 3.2 Hz, 3H), 2.14 (q, J = 7.9, 7.0 Hz, 2H), 1.90 (p, J = 7.0 Hz, 2H), 1.29 - 1.19 (m, 3H), 0.91 (t, J = 7.3 Hz, 3H), 0.57 - 0.37 (m, 4H).

### Compound 8: LC-MS results show [M+H]⁺: 534.2

### Example 7: Synthesis of Compound 9

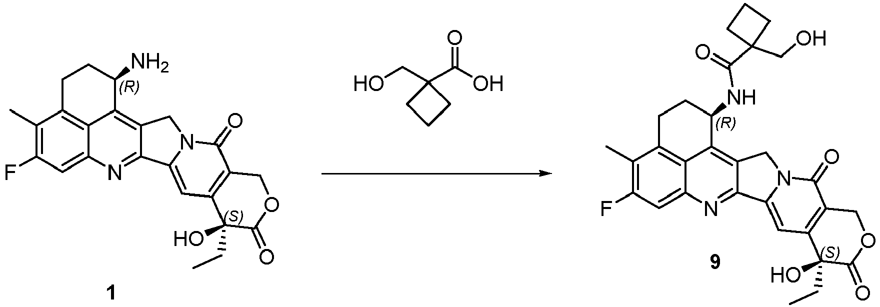

Compound **1** (in the form of trifluoroacetate) (100.0mg, 0.188mmol),1-(hydroxymethyl) cyclobutanic acid (43.0mg, 0.565mmol), HATU (107.2mg, 0.282mmol), HOBt(38.1mg,) and 5ml ultra-dry DMF was added to a 25ml round-bottomed flask with liquid seal at the mouth of the bottle. Stir the flask under a bath of ice water for 10 minutes, then add DIEA (94uL, 0.565mmol). The reaction progress was monitored by HPLC, and the reaction lasted for about 6h. After the reaction was complete, the reaction liquid was added to the saturated ammonium chloride aqueous solution to precipitate the solid, and the solid was extracted by extraction and filtration. The solid was washed twice, washed once with ethyl acetate, and washed once with methylene chloride. 100.0mg of solid Compound 9 was obtained with 98.0% yield.

LC-MS results show [M+H]⁺: 548.2.

¹H NMR (400 MHz, DMSO-d6) δ 7.99 (d, J = 8.6 Hz, 1H), 7.69 (d, J = 10.9 Hz, 1H), 7.29 (s, 1H), 6.52 (s, 1H), 5.53 (dt, J = 8.7, 5.5 Hz, 1H), 5.42 (s, 2H), 5.21 - 4.93 (m, 3H), 3.63 (d, J = 4.8 Hz, 2H), 3.10 (t, J = 6.2 Hz, 2H), 2.39 - 2.24 (m, 5H), 2.12 (q, J = 6.3 Hz, 2H), 1.99 - 1.70 (m, 6H), 0.91 (t, J = 7.3 Hz, 3H).

### Example 8: Synthesis of Compound M1

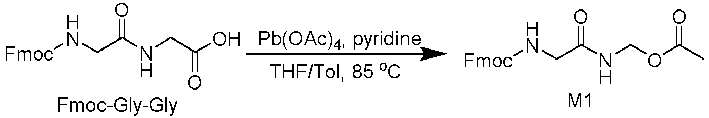

In a 5000 mL single-mouth bottle, n-fluorene methoxycarbonyl-glycine (100 g, 282 mmol, 1.0eq, purchased), lead tetraacetate (175 g, 553 mmol, 1.4 eq), 2000 mL dried tetrahydrofuran and 670 mL toluene were added, mixed evenly and protected by nitrogen. Heat to 85 °C for 2.5 h. TLC monitoring, after the reaction, the raw material was cooled to room temperature and filtered. The filtrate was decompressed and concentrated. The residue was purified by column chromatography to obtain compound M1(91 g). LC-MS: [M+NH₄] ⁺ 386.0.

### Example 9: Synthesis of Compound M2

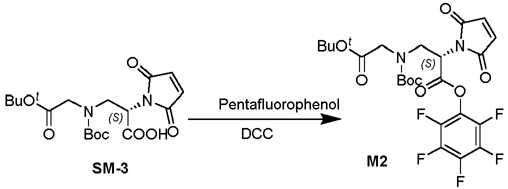

Compound SM-3 (49.9g, 100.0mmol, 1.0eq) was added into 1000 mL single-mouth bottle. Synthesis was performed by referring to the method disclosed in CN108452321, pentafluorophenol (18.5 g, 110.0mmol, 1.1eq), DCC (20.64 g, 110.0mmol, 1.1eq) and THF (500 mL) are reacetd at room temperature for 1h (TLC is used to monitor the complete reaction), filter to rid of insoluble matter, and record the filtrate as filtrate A, stored at 2-8°C for later use (if use directly, calculated at 0.2M). LC-MS: [M+H]⁺565.2_{∘}

### Example 10: Synthesis of Compound Ln-D1

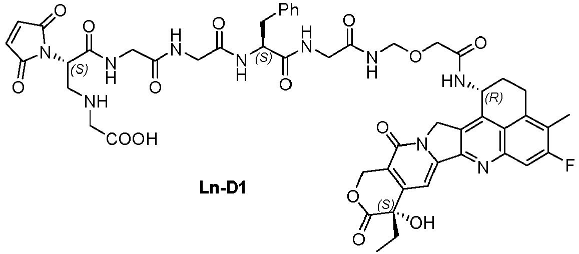

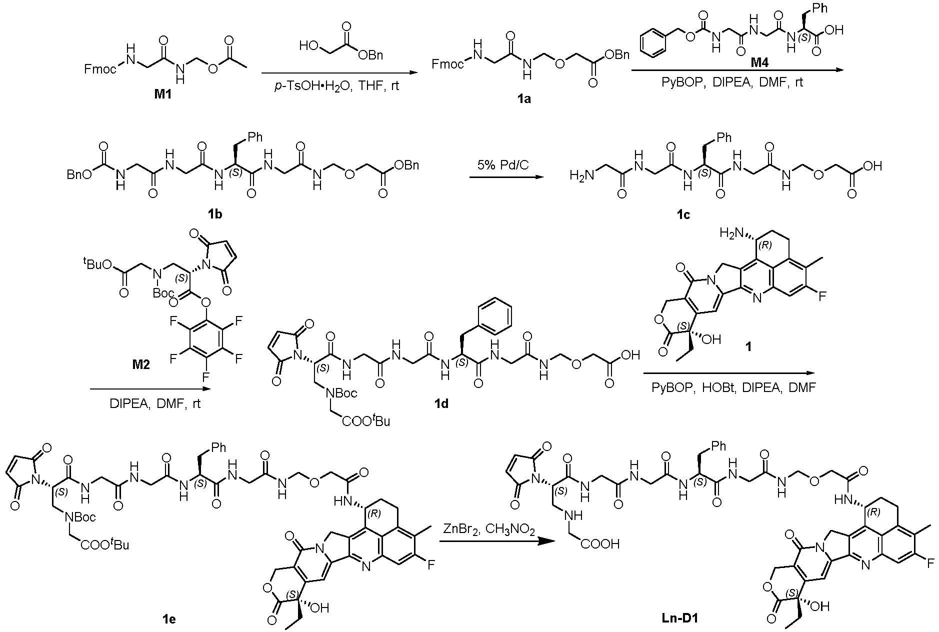

### Step 1: Synthesis of the Compound 1a

Add M1 (15.00g, 40.75mmol) and 150ml THF into a 500ml round-bottomed flask and stir under an ice bath for 15min. Add p-toluene sulfonic acid monohydrate (775.0mg, 4.08mmol) and continue stirring for 10min. A constant pressure drip funnel was installed at the upper end of the flask, and benzyl hydroxylacetate (11.6ml, 81.50mmol) was added. After the drip is finished, continue to stir under the ice bath, and the reaction is complete for about 3h. After the disappearance of the raw material detected by TLC, the mixture was quenched with saturated sodium bicarbonate, extracted with ethyl acetate for three times, dried with anhydrous sodium sulfate, concentrated and separated by silica gel column chromatography. 9.06g of white solid was isolated with a yield of 46.9%.

### Step 2: Synthesis of the Compound 1b

Add 1a (5.34g, 11.26mmol) and 25ml DMF into a 50ml round-bottoming flask No. 1 and stir under an ice bath for 15min. Triethylamine (1854uL, 12.39mmol) was drip-added, and the reaction was about 5h. TLC was used to detect whether the raw material disappeared, and the reaction was completed and put aside until use. Add M4 (4.65g, 11.25mmol), PyBop (7.03g, 13.52mmol), DIEA (2226uL, 13.56mmo) and 20ml DMF to a 100ml round bottomed flask No.2, and stir under an ice bath for 15min. Add the reaction dropwise from the fully reacted No. 1 bottle to the reaction No. 2 bottle. HPLC was used to monitor whether the reaction material disappeared. After the reaction was complete, HPLC was used for preparation and separation. The freeze-dried product was 4.83g white solid with yield of 66.3%.

### Step 3: Synthesis of the Compound 1c

1b (500.0mg, 0.772mmol), 5% Pd/C (500.0mg, 100% m) and 10ml DCM were added into a 50 mL single-mouth bottle, hydrogen balloons were added and hydrogen was replaced, and the reaction was about 3h at room temperature. The reaction was monitored by HPLC. Filtration was done to obtain filtrate, which is the crude product 1c to be used directly for the next reaction.

### Step 4: Synthesis of the Compound 1d

The crude product 1c was placed in ice water bath, DIPEA(0.14mL, 0.82 mmol) was added, compound M2(4.0mL, 0.8 mmol) was added, the temperature was raised to room temperature and let react for 1 h. After the reaction was monitored by HPLC and purified by liquid phase, 370.2mg white solid was obtained by freeze-drying with yield of 59.7%. LC-MS: [M+H]⁺ 804.4.

### Step 5: Synthesis of the Compound 1e

Add 1d (260.0mg, 0.281mmol), Compound 1 (149.3mg, 0.281mmol), HATU (160.4mg, 0.422mmol) ,HOBt (57.0mg, 0.422mmol) and 5ml DMF to a 10ml single-mouth bottle. After the reaction was completed as monitored by HPLC, 368.0mg yellow solid was prepared and separated. The preparation liquid was lyophilized and the yield was 97.3%. LC-MS: [M+H]⁺ 1221.5.

### Step 6: Synthesis of the Compound Ln-D1

Add 1e (368mg, 0.289mmol), zinc bromide (1301.0mg, 5.78mmol) and 15ml nitromethane into a 25ml single-mouth bottle and stir at room temperature. After the disappearance of raw material as monitored by HPLC, the reaction liquid was concentrated and purified by HPLC. The preparation liquid was lyophilized to obtain 120mg yellow solid with a yield of 38.5%. LC-MS: [M+H]⁺ 1065.4.

### Example 11: Synthesis of Compound Ln-D2

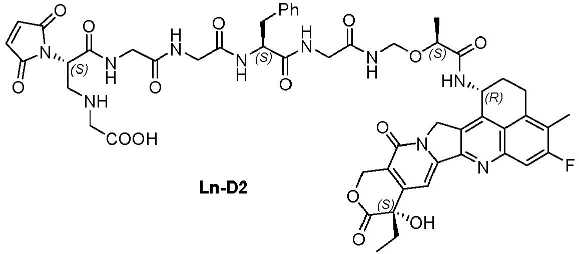

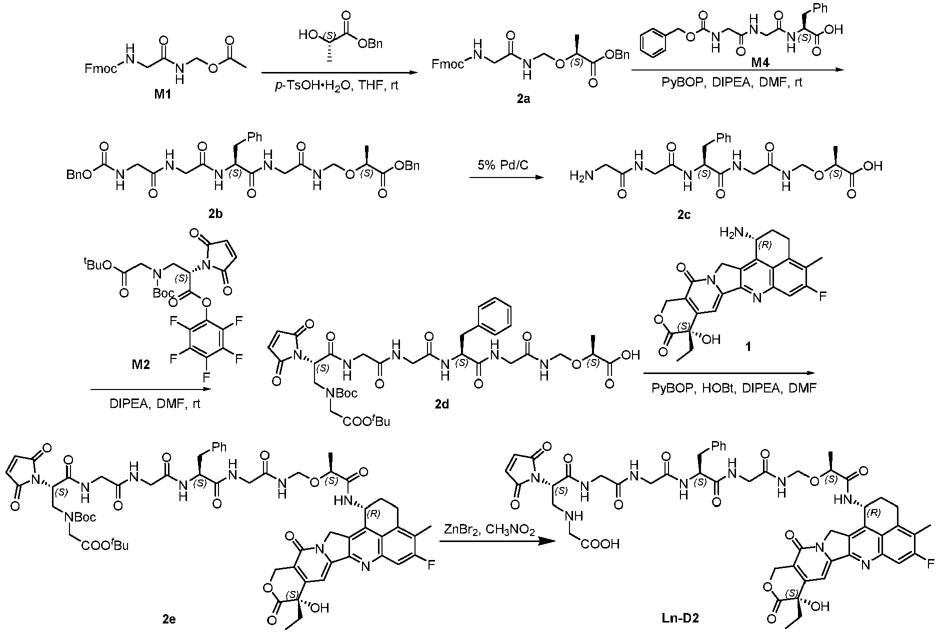

### Step 1: Synthesis of Compound 2a

Add M1 (7.45g, 20.3mmol) and 120ml THF into a 250ml round-bottomed flask and stir under an ice bath for 15min. Add p-toluene sulfonic acid monohydrate (385.0mg, 2.03mmol) and continue stirring for 10min. A constant pressure drip funnel was installed on the upper end of the flask, and benzyl hydroxylacetate (6.0ml, 41.5mmol) was added to the drops at a rate of 2 seconds. After the drip is finished, continue to stir under the ice bath, and the reaction is complete for about 3h. After the disappearance of the raw material detected by TLC, the mixture was quenched with saturated sodium bicarbonate, extracted with ethyl acetate for three times, dried with anhydrous sodium sulfate, concentrated and separated by silica gel column chromatography. 4.82 white solid was obtained with 49% yield Step 2: Synthesis of Compound **2b**

Add 2a (2.53g, 5.20mmol) and 10ml DMF to 25ml round-bottomed flask No. 1 and place under an ice bath for stirring for 15min. DBU (900uL, 6.0mmol) was added, and the reaction was about 0.5h. TLC was used to detect whether the raw material disappeared. M4 (2.31g, 5.6mmol), PyBop (3.50g, 6.8mmol), DIEA (1115 ul, 6.8mmo) and 10ml DMF were added to the No. 2 50ml round-bottomed flask, and stirred under an ice water bath for 15min. The reaction in the No. 1 flask were added dropwise to the No. 2 flask. HPLC was used to monitor whether the reaction material disappeared. After the reaction was complete, HPLC was used for preparation and separation. Lyophilize to obtain 2.53g white solid, 74% yield.

### Step 3: Synthesis of Compound 2c

2b (500.0mg, 0.76mmol), 5% Pd/C (500.0mg, 100% m) and 10ml DCM were added into a 50 mL single-mouth bottle. Hydrogen balloons were added and hydrogen was replaced. The reaction was carried out for about 3h at room temperature. The reaction was monitored by HPLC. The filtrate was obtained by filtration, and the crude product 2c was directly used in the next reaction.

### Step 4: Synthesis of Compound 2d

The crude product 2c was placed in an ice water bath, DIPEA (0.12mL, 0.70 mmol) was added, and compound M2 (4.0mL, 0.8 mmol) was added, and the mixture was raised to room temperature and let react for 1 h. After the reaction was monitored by HPLC and purified by liquid phase, 378.2mg white solid was obtained by lyophilization with a yield of 62%. LC-MS: M+H]⁺ 818.3.

### Step 5: Synthesis of Compound 2e

2d (228mg, 0.28mmol), Compound 1 (148.3mg, 0.28mmol), HATU (160.1mg, 0.42mmol), HOBt (57.2mg, 0.42mmol) and 5ml DMF were added to a 10ml single-mouth bottle and placed under an ice bath for stirring for 10min. DIEA (140uL, 0.84mmol) was added dropwise to continue the reaction. After the reaction was completed as monitored by HPLC, 269.5mg yellow solid was prepared and separated with yield 78%. LC-MS: [M+H]⁺ 1235.5.

### Step 6: Synthesis of Compound Ln-D2

Add 2e (269.5mg, 0.22mmol), zinc bromide (1012.9mg, 0.44mmol) and 10ml nitromethane to a 25ml single-mouth bottle and stir at room temperature. After the disappearance of raw material as monitored by HPLC, the reaction liquid was purified by HPLC after concentration, and the preparation liquid was lyophilized to obtain 96.3mg yellow solid with a yield of 41%. LC-MS: [M+H]⁺ 1079.4.

### Example 12: Synthesis of compound Ln-D3

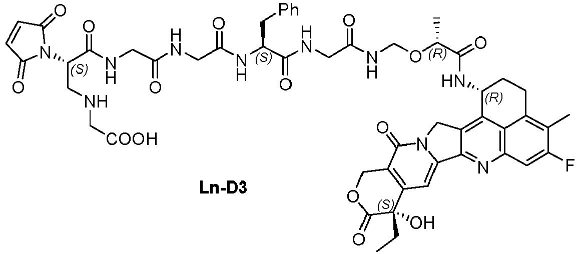

According to the synthesis route and method of Embodiment 11, compound L-D3(93.8mg) was obtained. LC-MS: [M+H]⁺ 1079.4.

### Example 13: Synthesis of compound Ln-D4 and Ln-D5

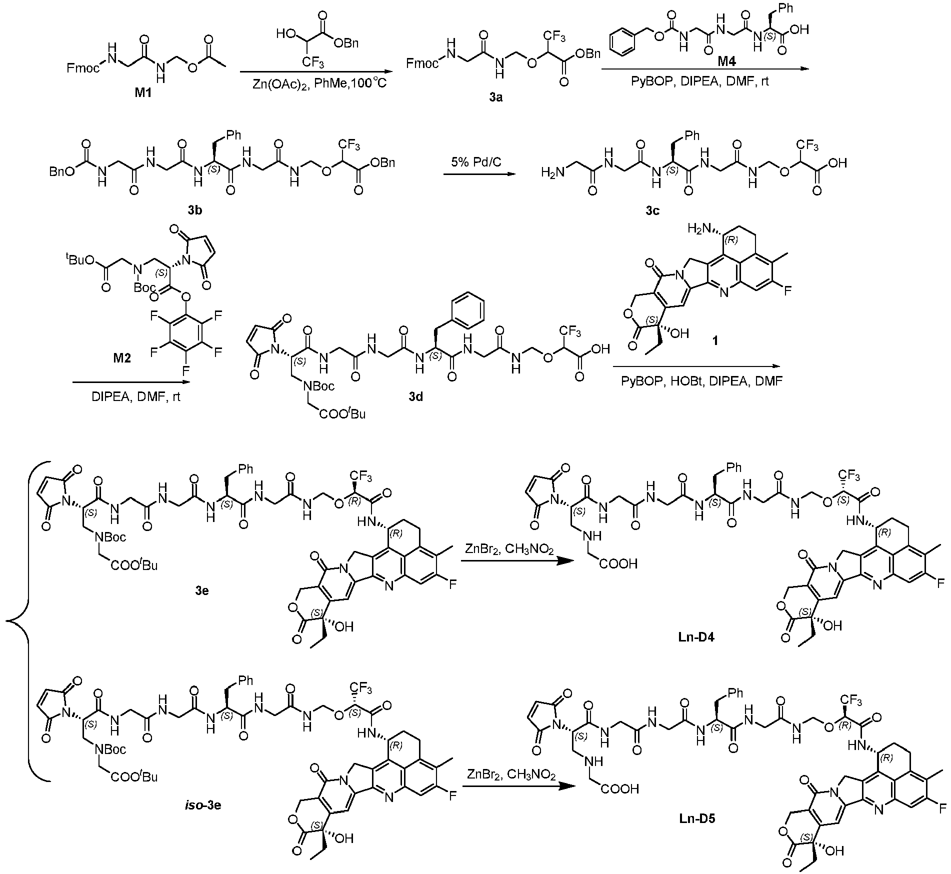

### Step 1: Synthesis of Compound 3a

M1 (2.87g, 7.79mmol), benzyl trifluoride lactate (3.65g, 15.59mmol), anhydrous zinc acetate (2.86g, 15.59mmol) and 30ml toluene were added to a 100ml round-bottomed flask. The reaction was carried out at 100°C under nitrogen protection. The reaction was carried out for about 5h under TLC monitoring. After the reaction is complete, the filtrate is concentrated into a crude oil. The crude oil was purified by silica gel column chromatography to obtain 1.51g white solid with yield of 35.7%.

### Step 2: Synthesis of Compound 3b

Add 3a (2.82g, 5.20mmol) and 10ml DMF to a 50ml round-bottomed flask No. 1, and stir under an ice bath for 15min. DB U(0.949g, 6.24mmol) was added dropwise, and the reaction lasted about 0.5h. TLC was used to detect whether the raw material disappeared. Add M4 ((2.36g, 5.71mmol), PyBop (3.25g, 6.24mmol), HOBt (0.84g, 6.24mmol), DIEA (1000uL, 6.24mmol) and 10ml DMF to a 50ml round-bottomed flask No.2. Stir in the ice bath for 15min, and add the reaction droplets in the reaction bottle No. 1 to the reaction bottle No. 2. HPLC was used to monitor whether the reaction material disappeared. After the reaction was complete, HPLC was used for preparation and separation. The prepared liquid was extracted by DCM for 3 times, dried with anhydrous sodium sulfate, concentrated organic phase, and vacuum dried to obtain 2.32g white solid with yield of 62%.

### Step 3: Synthesis of Compound 3c

3b (500.0mg, 0.70mmol), 5% Pd/C (500.0mg, 100% m) and 10ml DMF were added into a 50 mL single-mouth bottle and hydrogenated for about 3h at room temperature. The reaction was monitored by HPLC. Filtration was carried out to obtain filtrate, which is the crude product 3c to be used directly for the next reaction.

### Step 4: Synthesis of Compound 3d

The crude product 3c was placed in ice water bath, DIPEA (0.12mL, 0.70 mmol) was added, compound M2 (4.0mL, 0.8 mmol) was added, and the temperature was raised to room temperature to let react for 1 h. After the reaction was completed as monitored by HPLC, liquid phase was purified by lyophilization to obtain 280.0mg white solid with a yield of 46%. LC-MS: [M+H]⁺ 872.3.

### Step 5: Synthesis of Compound 3e

Add **3d** (280.0mg, 0.241mmol), Compound 1 (142.2mg, 0.241mmol), HATU (137.6mg, 0.362mmol), HOBt (38.9mg, 0.362mmol) and 5ml DMF to a 10ml single-mouth bottle. DIEA (120uL, 0.723mmol) was added dropwise to continue the reaction. After the reaction was cpmpleted as monitored by HPLC, the preparation was prepared and separated. The preparation solution was lyophilized to obtain 154.1mg yellow solid 3e with 73.6% yield, LC-MS: [M+H]⁺ 1289.5, and 62.5mg yellow solid iso-3e with 29.8%, LC-MS: [M+H]⁺ 1289.5.

### Step 6: Synthesis of Compound Ln-D4 and Ln-D5

Add **3e** (154.1mg, 0.118mmol), zinc bromide (532.5mg, 2.36mmol) and 10ml nitromethane into a 25ml single-mouth bottle and stir at room temperature. After the disappearance of raw material was monitored by HPLC, the reaction liquid was purified by HPLC after concentration, and the preparation liquid was lyophilized to obtain 82.0mg yellow solid with yield of 60%. LC-MS: [M+H]⁺ 1133.4.

Add iso-3e (62.5mg, 0.048mmol), zinc bromide (216.0mg, 0.959mmol) and 7ml nitromethane into a 25ml single-mouth bottle and stir at room temperature. After the disappearance of raw material was monitored by HPLC, the reaction liquid was purified by HPLC after concentration, and the preparation liquid was lyophilized to obtain 96.3mg yellow solid with a yield of 41%. LC-MS: [M+H]⁺ 1133.4.

### Example 14: Synthesis of compounds Ln-D6 and Ln-D7

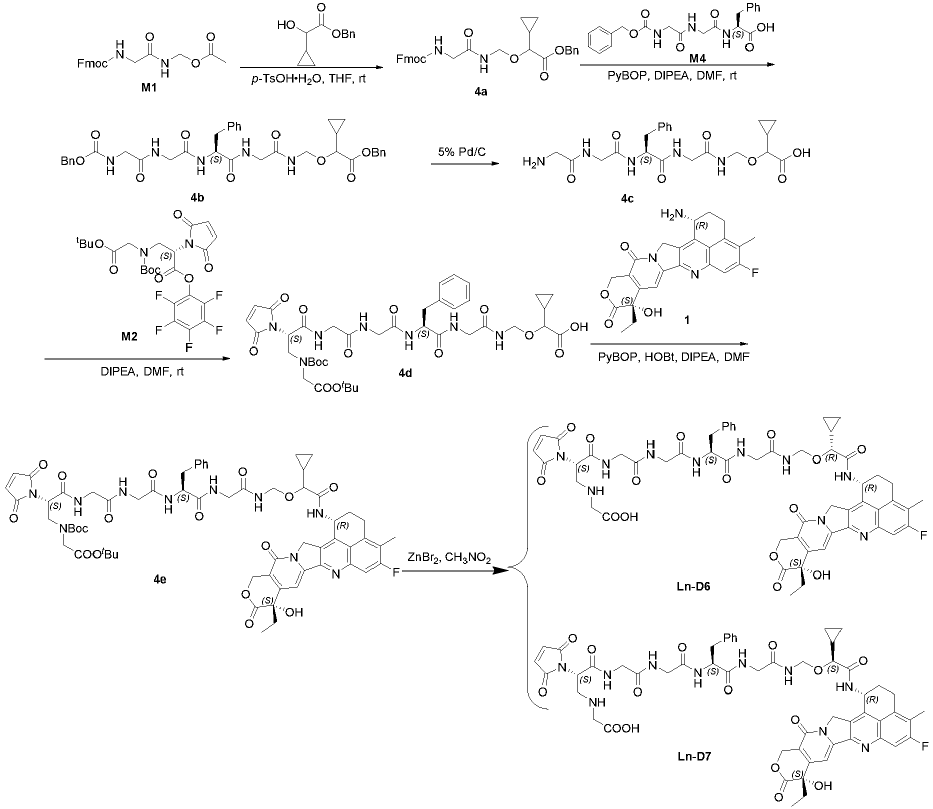

### Step 1: Synthesis of Compound 4a

Add M1 (3.75g, 10.19 mmol) and 50 ml THF to a 250 ml round bottom flask and stir under an ice water bath for 15 min. Add p-toluenesulfonic acid monohydrate (194.0mg, 1.02mmol) and continue stirring for 10min. A constant pressure drip funnel was installed at the upper end of the flask, 2-cyclopropyl-2-hydroxyacetate benzyl ester (4.34g, 20.4mmol) was added dropwise, the reaction was completed in about 6 h. After the TLC detection verifying that the raw material disappeared, the reaction was quenched with saturated sodium bicarbonate, the mixture was extracted with ethyl acetate 3 times, dehydrated with anhydrous sodium sulfate, and separated by silica gel column chromatography. 2.13g of white solid was with a yield of 41%.

### Step 2: Synthesis of Compound 4b

Add 4a (2.80g, 5.38mmol) and 10ml DMF to a 50ml round-bottomed flask No. 1, and stir the mixture for 15min in an ice bath. DIEA(0.981g, 6.45mmol) was added dropwise, and the reaction was about 0.5h. TLC was used to detect whether the raw material disappeared. Add M4 (2.22g, 5.38mmol), PyBop (3.35g, 6.45mmol), HOBt (0.87g, 6.45mmol), DIEA (1000uL, 6.45mmol) and 10ml DMF to a 100ml round bottling flask No.2. Stir in the ice bath for 15min, and add the reaction from the reaction bottle No. 1 dropwise to the reaction bottle No. 2. HPLC was used to monitor whether the reaction material disappeared. After the reaction was complete, HPLC was used for preparation and separation. 2.63g white solid was obtained by lyophilization at 61% yield.

### Step 3: Synthesis of Compound 4c

4b (1003.1mg, 1.46mmol), 5% Pd/C (1004.2mg, 100% m) and 15ml DMF were added into a 50 mL single-mouth bottle. Hydrogen balloon was added and Hydrogen gas was replaced and let react at room temperature for about 3h. The reaction was monitored by HPLC. Filtration was carried out to obtain filtrate, i.e., crude product 4c for use for the next reaction.

### Step 4: Synthesis of Compound 4d

The crude product 4c was placed in ice water bath, DIPEA (0.26mL, 1.6mmol) was added, and compound M2 (8.0mL, 1.6mmol) was added. The mixture was raised to room temperature and react for 2 h. After the reaction was completed as monitored by HPLC, purified in liquid phase, the prepared liquid was obtained and lyophilized to obtain 945.1mg white solid with a yield of 76%.

The crude product 4c was placed in ice water bath, DIPEA(0.26mL, 1.6mmol) was added, and compound M2(8.0mL, 1.6mmol) was added. The mixture was raised to room temperature and reacted for 2 h. After the reaction was completed as monitored by HPLC, liquid phase was purified and lyophilized to obtain 945.1mg white solid with a yield of 76%. LC-MS: [M+H]⁺ 844.4.

### Step 5: Synthesis of Compound 4e

Add 4d (422.0mg, 0.50mmol), Compound 1 (265.5mg, 0.50mmol), HATU (285.1mg, 0.75mmol), HOBt (101.3mg, 0.75mmol) and 5ml DMF to a 10ml single-mouth bottle. Stir under ice bath for 10min. DIEA (248uL, 1.50mmol) was added dropwise to continue the reaction. After the reaction was completed as monitored by HPLC, the preparation was prepared and separated. The preparation solution was lyophilised to 268.0mg yellow solid with a yield of 42%. LC-MS: [M+H]⁺ 1261.5.

### Step 6: Synthesis of Compounds Ln-D6 and Ln-D7

Add 4d (268.0mg, 0.204mmol), zinc bromide (1168.1mg, 5.187mmol) and 10ml nitromethane into a 50ml single-mouth bottle and stir at room temperature. After the reaction was completed as modified by HPLC, the reaction liquid was concentrated first and then purified by HPLC, and the preparation liquid was lyophilized to obtain 68.0mg Ln-D6 yellow solid with a yield of 28% and LC-MS: [M+H]⁺ 1105.4, and 82.0mg Ln-D7 yellow solid with a 35% yield and LC-MS: [M+H]⁺ 1105.4.

### Example 15: Synthesis of Compound Ln-D8

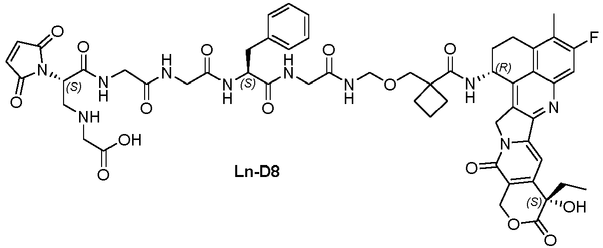

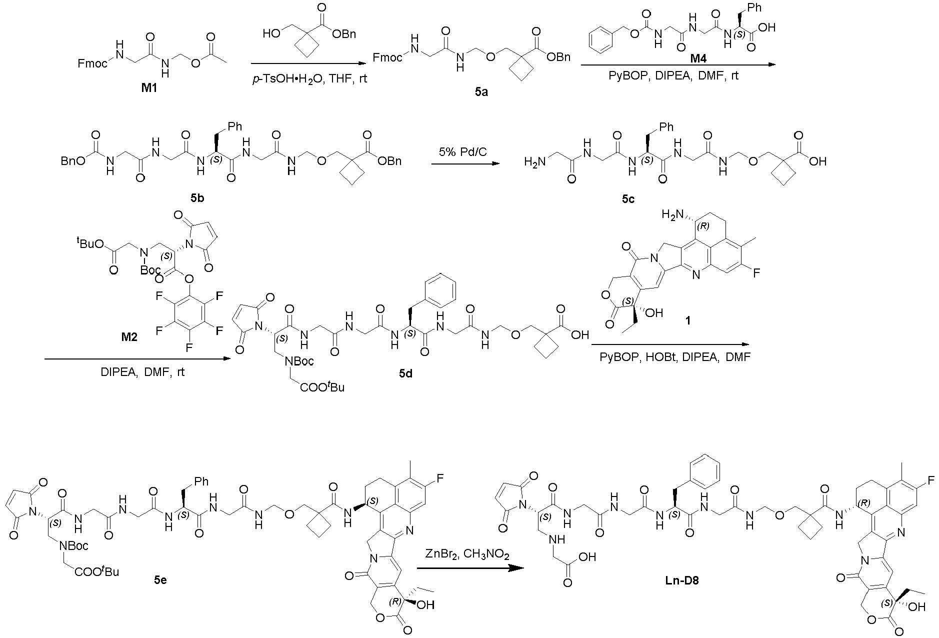

### Step 1: Synthesis of Compound 5a

Add M1 (3.74g, 10. 16mmol) and 50ml THF into a 250ml round-bottomed flask and stir under an ice bath for 15min. Add p-toluene sulfonic acid monohydrate (193.1mg, 1.02mmol) and continue stirring for 10min. A constant pressure drip funnel was installed at the upper end of the flask, and 1-(hydroxymethyl) benzyl cyclobutanate (4.59g, 20.3mmol) was added in droplets. After the drip is finished, continue to stir under the ice bath, about 5h reaction is complete. After the disappearance of the raw material detected by TLC, the mixture was quenched with saturated sodium bicarbonate, extracted with ethyl acetate for three times, dried with anhydrous sodium sulfate, concentrated and separated by silica gel column chromatography. 4.60g of white solid was isolated and the yield was 82%.

### Step 2: Synthesis of Compound 5b

Add 5a (4.38g, 8.29mmol) and 20ml DMF into a 50ml round-bottomed flask No. 1 and place them under an ice bath for 15min. DBU (1363uL, 9.11mmol) was added dropwise, the reaction was about 0.5h, TLC was used to detect whether the raw material disappeared, and the reaction was completed. Add M4 (3.42g, 8.29mmol), PyBop (5.17g, 9.95mmol), HOBt (1.34g, 9.95mmol), DIEA (1640uL, 9.95mmol) and 30ml DMF to a 50ml round-bottom flask No.2. Stir in the ice bath for 15min, and add reaction from the reaction bottle No. 1 to the reaction bottle No. 2 by droplets. HPLC was used to monitor whether the reaction material disappeared. After the reaction was complete, HPLC was used for preparation and separation. After lyophilization, 4.60g white solids was obtained with 68% yield.

### Step 3: Synthesis of Compound 5c

5b (1041.5mg, 1.26mmol), 5% Pd/C (1056.7mg, 100% m) and 10ml DMF were added to a 50 mL single-mouth bottle, hydrogen balloon was added and hydrogen was replaced, and the reaction was about 3h at room temperature. After the reaction was completed as monitored by HPLC, the hydrogen was removed by ultrasound. Filtration was carried to obtain filtrate, i.e., crude product 5c, which was used directly for the next reaction.

### Step 4: Synthesis of Compound 5d

The crude product 5c was placed in ice water bath, DIPEA (0.24mL, 1.4mmol) was added, compound M2(7.0mL, 1.4mmol) was added, and the temperature was raised to room temperature to react for 1 h. After the reaction was completed as monitored by HPLC and the liquid phase was purified, 803.5mg white solid was obtained by lyophilization with yield of 64%. LC-MS: [M+H]⁺ 858.4.

### Step 5: Synthesis of Compound 5e

5d (201.3mg, 0.203mmol), M2 amino isomerism (100.0mg, 0.203mmol), HATU (107.4mg, 0.304mmol), HOBt (38.2mg, 0.304mmol) and 5ml DMF were added into a 10ml single-mouth bottle. Stir in the ice bath for 10 minutes. DIEA (248uL, 1.50mmol) was added to continue the reaction. After the reaction was completed as monitored by HPLC, the liquid phase was purified. The preparation liquid was lyophilized and the yield was 84%. LC-MS: [M+H]⁺ 1275.5.

### Step 6: Synthesis of Compound Ln-D8

Add 5d (219.1mg, 0.170mmol), zinc bromide (765.3mg, 3.40mmol) and 20ml nitromethane into a 50ml single-mouth bottle and stir at room temperature. After the reaction was completed as modified by HPLC, the reaction liquid was prepared and purified by HPLC, and the preparation liquid was lyophilized to obtain 120.2mg yellow solid with yield of 62%. LC-MS: [M+H]⁺ 1119.4.

### Example 16: Synthesis of Compound Ln-D9

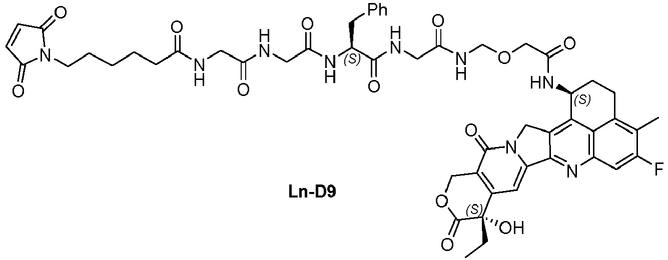

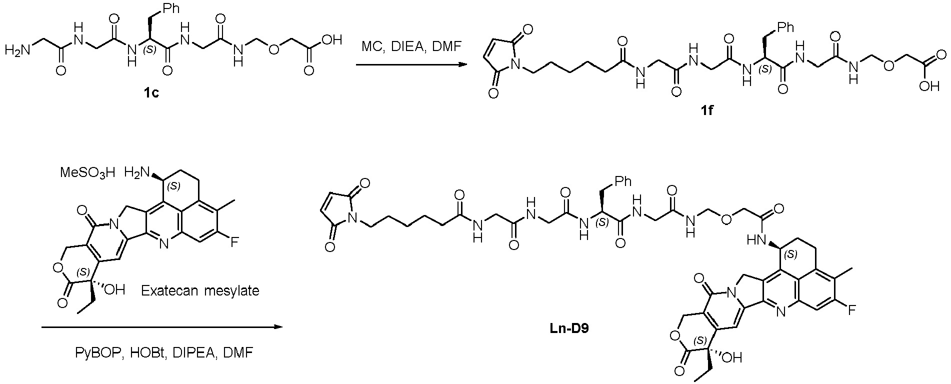

### Step 1: Synthesis of Compound 1f

1b (500.0mg, 0.772mmol), 5% Pd/C (500.0mg, 100% m) and 10ml DMF were added into a 50 mL single-mouth bottle. Hydrogen balloons were added and hydrogen gas replacement was done, and the reaction was carried out for about 3h at room temperature. After the reaction was completed as monitored by HPLC, the hydrogen was removed by ultrasound. The filtration was carried out to obtain filtrate and crude product 1c. Under the ice water bath, MC (280.1mg, 0.9 mmol), DIEA (235mg, 1.8mmol) were added into the filtrate successively, protected by nitrogen. After the addition, the filtrate was raised to room temperature to react for 1h, monitored by HPLC, prepare and purify liquid phase, and lyophilized to obtain compound 1f (268.9mg, 86%), MS: [M+H]⁺617 .2.

### Step 2: Synthesis of Compound Ln-D9

Add 1f (268.9mg, 0.44mmol), exatecan mesylate (233.8mg, 0.44mmol, purchased), HATU (190.5mg, 0.50mmol), HOBt (67.8mg, 0.50mmol), and 5ml DMF to a 10ml single-mouth bottle. Stir in the ice bath for 10 minutes. DIEA (140uL, 0s.84mmol) was added to continue the reaction. After the reaction was completed as monitored by HPLC, it was prepared and separated. The preparation solution was lyophilized to 254.3mg yellow solid with yield of 56%. LC-MS: [M+H]⁺ 1034.4.

### Example 17: Synthesis of compounds for control

The following compounds were synthesized by referring to the methods disclosed in patents CN111689980 and WO2020063676:

| **Compound numbering** | **Compound structure** |
|---|---|
| **10** | 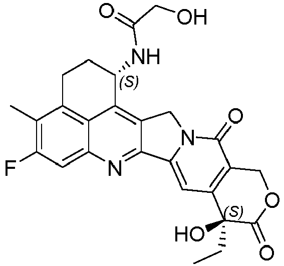 |
| **11** | 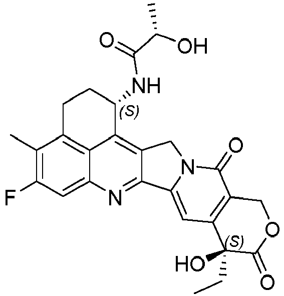 |
| **12** | 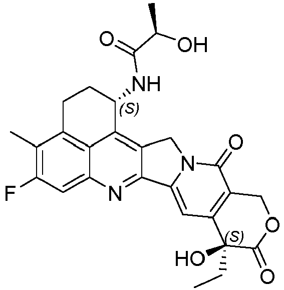 |
| **13** | 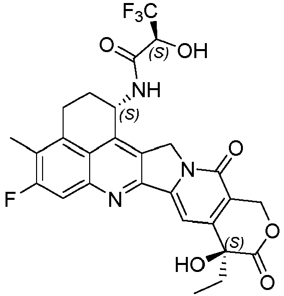 |
| **14** | 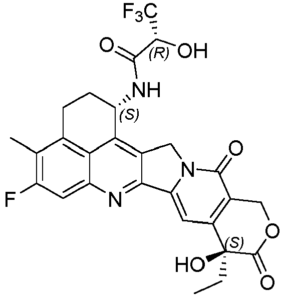 |
| **15** | 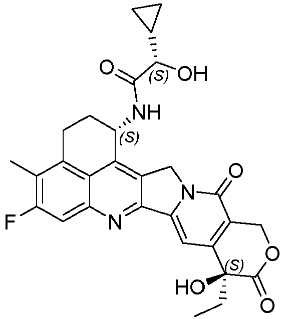 |
| **16** | 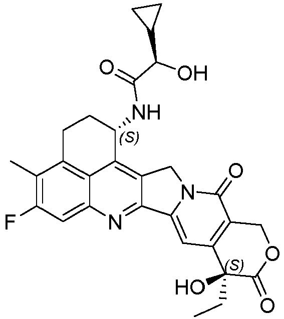 |
| **17** | 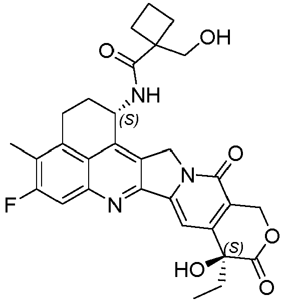 |

### Example 18: General methods for coupling and preparing ADC conjugates

An antibody Ab with a monomer ratio greater than 95% after preliminary purification was transferred to the phosphate buffer with a concentration of 10mg/ml using an ultrafiltration centrifuge tube. TCEP (20 times the number of molar molecules of antibody) was added, and the reaction was performed for 10h at room temperature to open the disulfide bond between antibody chains. A linker-toxin was added with 20 times the number of molar molecules of antibody, and the reaction was conducted for 2h at room temperature. After the reaction, the ultrafiltration centrifuge tube with the interception molecular weight of 30KDa was used to change the liquid into PBS, and the uncoupled payload was removed. ADC samples after liquid change are filtered by a 0.22 micron bacteria-removing filter.

Payload Ln-D1, Ln-D2, Ln-D4, Ln-D5, Ln-D6, Ln-D7, Ln-D8 and Ln-D9 are coupled with antibody molecule Ab using the coupling method described in this embodiment 18 (Antibody molecule Ab can be A: Trastuzumab; B: Cetuximab). The average drug/antibody ratio (DAR) of the conjugate products was determined by reversed-phase high performance liquid chromatography. The obtained information about ADC drug molecules and the corresponding information with payload molecule are as follows:

| **Payload numbering** | **Coupling product ADC numbering** | **Antibody Ab** | **ADC Structure** | **DAR** |
|---|---|---|---|---|
| **Ln-D9** | **ADC-1** | A | 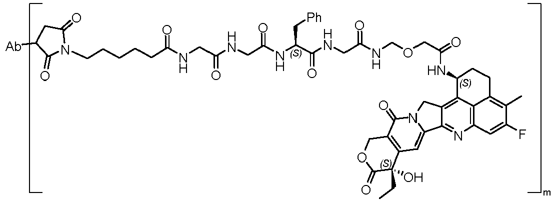 | 7.7 |
| **Ln-D1** | **ADC-2** | A | 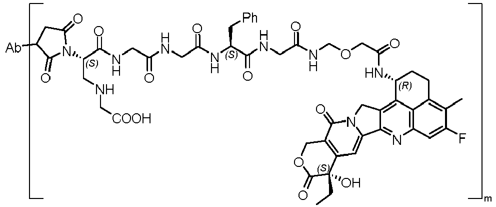 | 7.4 |
| **Ln-D2** | **ADC-3** | A | 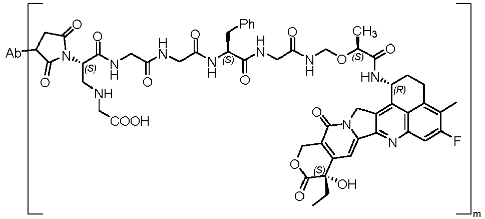 | 7.5 |
| **Ln-D4** | **ADC-4** | A | 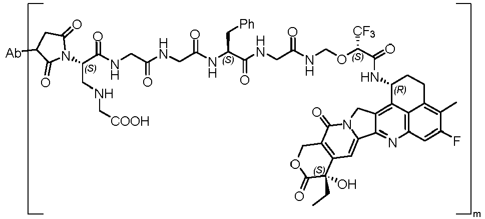 | 7.3 |
| **Ln-D5** | **ADC-5** | A | 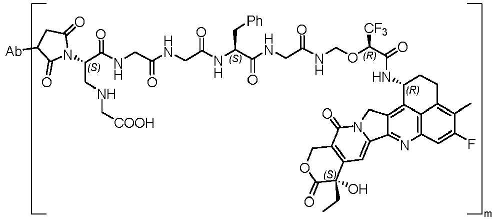 | 7.5 |
| **Ln-D6** | **ADC-6** | A | 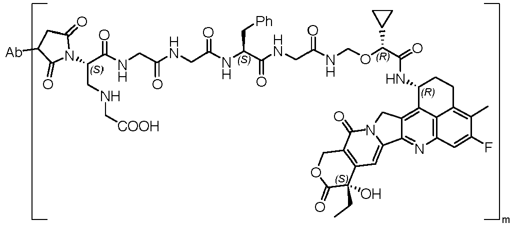 | 7.5 |
| **Ln-D7** | **ADC-7** | A | 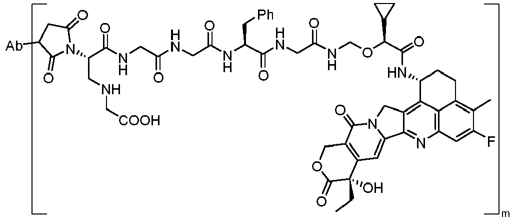 | 7.5 |
| **Ln-D8** | **ADC-8** | A | 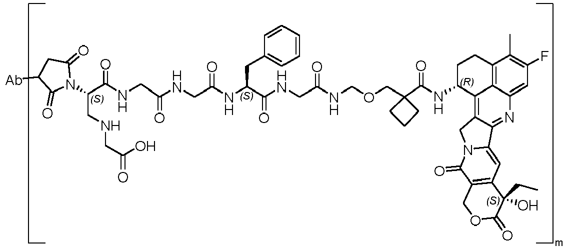 | 7.7 |
| **Ln-D2** | **ADC-9** | B | 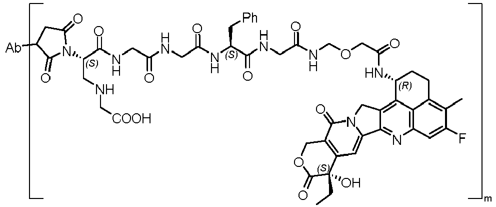 | 7.3 |
| **Ln-D7** | **ADC-10** | B | 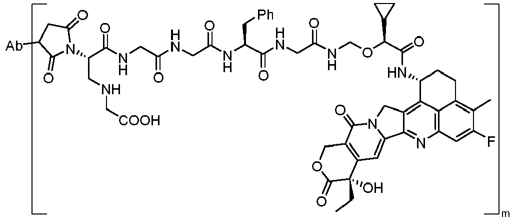 | 7.4 |

### Example 19: The logP value test for chiral derivatives of camptothecin

The logP values are closely related to water solubility, membrane permeability, in vivo ADME process and affinity between the compound and the receptor, which play a key role in the process of the compound's penetration through the biofilm. The logP value was obtained by testing the partition coefficient of camptothecin drug in n-octane (oil) and water, the results are shown as follows:

| **Camptothecin derivatives numbering** | **Camptothecin derivative structural formula** | **LogP** |
|---|---|---|
| **2** | 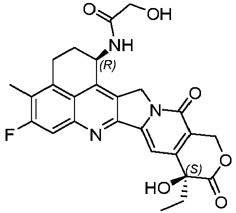 | 1.8 |
| **3** | 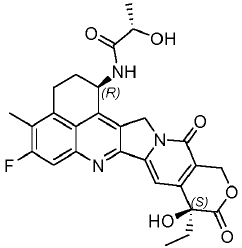 | 1.6 |
| **4** | 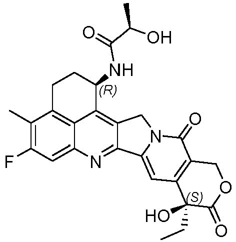 | 1.9 |
| **5** | 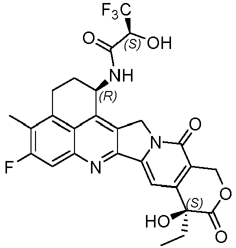 | 1.9 |
| **6** | 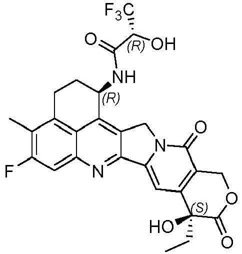 | 1.9 |
| **7** | 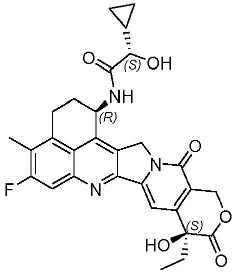 | 1.7 |
| **8** | 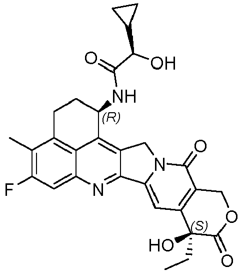 | 1.8 |
| **9** | 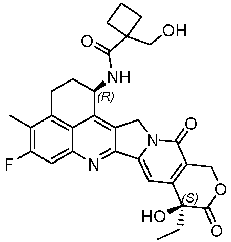 | 1.6 |
| **10** | 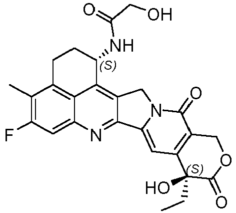 | 2.3 |
| **11** | 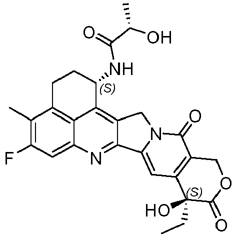 | 2.5 |
| **12** | 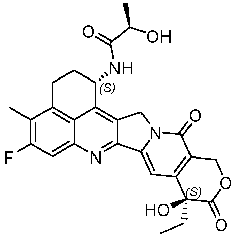 | 2.5 |
| **13** | 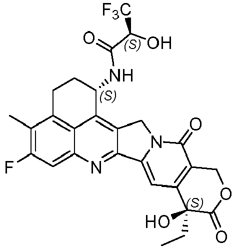 | 2.6 |
| **14** | 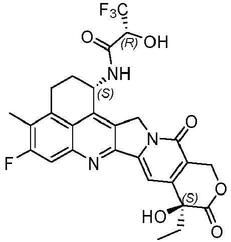 | 2.6 |
| **15** | 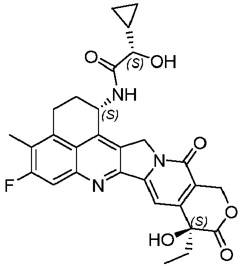 | 2.7 |
| 16 | 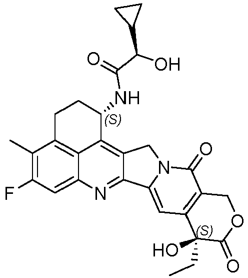 | 2.6 |
| 17 | 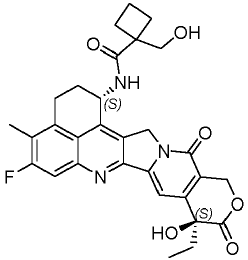 | 3.0 |

The above LogP test values show that the compound of the instant invention has lower LogP value and better water solubility.

### Example 20: Testing plasma aggregation and degradation of ADC

Aseptic ADC samples were taken and added into human plasma from which human IgG had been removed, so that the final concentration of ADC in plasma was 0.6mg/ml. Three tubes of each type of ADC were prepared and incubated in a water bath at 37°C for 0h, 72h and 7d, respectively. ADC samples were taken out. 100µL ProteinA (MabSelect SuRe TMLX Lot:#10221479GE, washed with PBS) was added to each tube, and the vertical mixer was shaken and adsorbed for 2h. After washing, elution and Tris-HCl neutralization, the incubated ADC was obtained. SEC detection was performed on ADC samples incubated for a specific time to determine aggregation and degradation.

| **ADC numbering** | **Condition** | **HMW**%/ aggregation% | **LMW**%/ degradation% |
|---|---|---|---|
| **ADC-1** | unincubated | 2.05 | 2.67 |
| | Plasma incubation 0D | 48.43 | 5.39 |
| | Plasma incubation 3D | 46.84 | 3.55 |
| | Plasma incubation 7D | 54.82 | 2.29 |
| **ADC-2** | unincubated | 8.18 | 2.34 |
| | Plasma incubation 0D | 26.36 | 1.98 |
| | Plasma incubation 3D | 25.16 | 1.72 |
| | Plasma incubation 7D | 26.75 | 0.86 |
| **ADC-3** | unincubated | 4.41 | 2.44 |
| | Plasma incubation 0D | 28.69 | 1.98 |
| | Plasma incubation 3D | 28.89 | 1.50 |
| | Plasma incubation 7D | 28.94 | 1.11 |
| **ADC-4** | unincubated | 4.01 | 2.53 |
| | Plasma incubation 0D | 27.65 | 3.03 |
| | Plasma incubation 3D | 27.70 | 2.35 |
| | Plasma incubation 7D | 27.96 | 1.24 |
| **ADC-5** | unincubated | 2.28 | 2.35 |
| | Plasma incubation 0D | 20.68 | 1.43 |
| | Plasma incubation 3D | 16.60 | 0.96 |
| | Plasma incubation 7D | 21.25 | 0.63 |
| **ADC-6** | unincubated | 2.90 | 2.49 |
| | Plasma incubation 0D | 28.59 | 2.04 |
| | Plasma incubation 3D | 26.16 | 1.72 |
| | Plasma incubation 7D | 26.17 | 0.90 |
| **ADC-7** | unincubated | 3.12 | 2.62 |
| | Plasma incubation 0D | 28.97 | 2.23 |
| | Plasma incubation 3D | 31.02 | 1.53 |
| | Plasma incubation 7D | 32.32 | 0.46 |
| **ADC-8** | unincubated | 3.08 | 2.49 |
| | Plasma incubation 0D | 25.94 | 1.77 |
| | Plasma incubation 3D | 26.52 | 1.22 |
| | Plasma incubation 7D | 29.90 | 1.69 |
| **ADC-9** | unincubated | 2.16 | 2.76 |
| | Plasma incubation 0D | 18.43 | 1.39 |
| | Plasma incubation 3D | 22.48 | 1.92 |
| | Plasma incubation 7D | 24.28 | 1.55 |
| **ADC-10** | unincubated | 3.27 | 2.38 |
| | Plasma incubation 0D | 21.34 | 1.93 |
| | Plasma incubation 3D | 26.48 | 1.78 |
| | Plasma incubation 7D | 25.83 | 2.03 |

The payload of ADC-1 is the same as that of the listed antitumor drug Enhertu. From the test experiments of ADC drug aggregation and degradation in plasma, it can be seen that the aggregation and degradation of ADC derived from a chiral derivative of camptothecin is significantly lower than those of ADC-1, ADC derived from a chiral derivative of camptothecin has better physical and chemical properties and can exist more stably in plasma compared to ADC-1.

### Example 21: Cell activity test of chiral derivatives of camptothecin

The cytotoxic activity of camptothecin chiral derivatives was determined by the following experimental procedure: the chiral derivatives of camptothecin were added to A431, MDA-MB-468, SK-BR-3, Bxpc-3 and SW620 tumor cells, respectively, and the cell survival rate was determined 72 hours later. Cell-based in vitro experiments are used to determine cell survival, cytotoxicity and programmed cell death induced by camptothecin drugs disclosed in the present invention.

The *in vitro* efficacy of camptothecin was determined by cell proliferation test. CellTiter 96^{®} Aqueous One Solution Cell Proliferation Assay is commercially available (Promega Corp., Madison, WI). CellTiter96^{®}Aqueous One Solution Cell Proliferation Assay(a) is a test reagent that can be used to measure the number of viable cells from cell proliferation and cytotoxicity tests by colorimetric assay. The reagent contains a new tetrazolium compound [3-(4, 5-dimethylthiazol-2-yl)-5-(3-carboxymethoxyphenyl)-2-(4-sulfophenyl) -2h-Tetrazolium,inner salt; MTS] and an electron coupling agent (phenazine ethosulfate; PES). PES has enhanced chemical stability, which allows it to be mixed with MTS to form stable solutions. MTS (Owen's reagent) is reduced by cellular organisms into a variety of colored methylate products, which can be dissolved directly in the medium. This transformation is most likely accomplished with the help of NADPH or NADH produced by dehydrogenase in metabolically active cells. For detection, appropriate amount of CellTiter96^{®}Aqueous One Solution Reagent is added directly into the medium of culture plate hole, incubated for 1-4 hours, and the absorbance value of 490nm is read with enzyme label instrument.

The amount of methylate detected at 490nm is proportional to the number of live cells in the culture. Due to the dissolubility of the methyls of MTS in tissue medium, CellTiter 96^{®}Aqueous One Solution is performed with fewer steps than the MTT or INT methods.

In the invention, A431, MDA-MB-468, SK-BR-3, Bxpc-3 and SW620 are used as the research system of in vitro efficacy detection. The 96-well plates were uniformly inoculated with appropriate cell density and incubated at 37°C in carbon dioxide incubator. 24 hours later, the cells were confirmed to be normal under the microscope, and camptothecin drugs were administered. Camptothecin drug was diluted in the medium (initial concentration was 1uM, 7 times diluted, with 8 concentration points. The last two columns were control group (i.e., cell + medium, no drug treatment) and blank group (i.e., cell-free, only containing medium, for background subtraction). After fully mixing the drug, the drug was added into the corresponding cell hole and incubated at 37°C in a carbon dioxide incubator for 3 days. After 3 days, 20 ul MTS (Promegm, G3581) were added to each well for 2 h, and a machine reader (from Molecular Device, model: SpectraMAX190) took readings at 490nM. By detecting the activity of dehydrogenase in mitochondria, IC50 was calculated to evaluate the inhibitory effect of camptothecin on cell proliferation. The corresponding test results of camptothecin chiral derivative IC50 (nM) were as follows:

| **Cell lines** | **Compound numbering** | | | | | | | | |
|---|---|---|---|---|---|---|---|---|---|
| | **Dxd (purchased)** | 2 | 3 | 4 | 5 | 6 | 7 | 8 | 9 |
| **A431** | 4.991 | 4.496 | 4.188 | 3.104 | 2.829 | 0.689 | 0.961 | 1.841 | 1.516 |
| **BxPc-3** | 3.966 | 3.995 | 3.659 | 2.848 | 1.729 | 0.416 | 0.801 | 1.101 | 1.070 |
| **SW620** | 1.954 | 2.889 | 1.290 | 0.920 | 0.866 | 0.093 | 0.894 | 1.178 | 1.112 |
| **MDA-MB-468** | 10.653 | 0.002 | 0.22 | - | 8.913 | 2.79 | <0.001 | <0.001 | 0.525 |
| **SK-BR-3** | 19.72 | 1.78 | 14.982 | - | 8.49 | 9.10 | <0.01 | <0.01 | 7.32 |

Dxd is the active ingredient of cancer ADC drug Enhertu, which is a highly active camptothecin drug. Through cell activity experiments, the inventor proved that the chiral derivatives of camptothecin in the present invention showed equal or higher cell activity than Dxd in representative tumor cells BXPC-3, A431, SW620, MDA-MB-468 and SK-BR-3.

### Example 22: The in vitro plasma stability of ADC

Aseptic ADC samples were taken and added into sterile human plasma from which human IgG had been removed, so that the final concentration of ADC was 0.6mg/ml. Three tubes of each type of ADC were prepared and incubated in a water bath at 37°C for 0h, 72h and 7d, respectively, and ADC samples were taken out. 100µL ProteinA (MabSelect SuRe TMLX Lot:#10221479GE, washed with PBS) was added to each tube, and the vertical mixer was shaken and adsorbed for 2h. After washing, elution and Tris-HCl neutralization, the incubated ADC was obtained. DAR values of ADC samples incubated for a specific time were measured by RP-HPLC to determine the plasma stability of the samples. The experimental results show that the ADCs disclosed by the instant invention have little or no loss in the plasma incubation process, and has good stability in the plasma, because the possibility of toxicity caused by early degradation is low.

| **ADC-numbering** | **ADC-1** | **ADC-2** | **ADC-3** | **ADC-4** | **ADC-5** | **ADC-6** | **ADC-7** | **ADC-8** | **ADC-9** | **ADC-10** |
|---|---|---|---|---|---|---|---|---|---|---|
| **0 h (DAR)** | 7.51 | 7.44 | 7.29 | 7.42 | 7.49 | 7.47 | 7.52 | 7.62 | 7.39 | 7.42 |
| **72 h (DAR)** | 6.86 | 7.36 | 7.10 | 7.33 | 7.57 | 7.41 | 7.37 | 7.53 | 7.45 | 7.36 |
| **7d (DAR)** | 6.76 | 7.30 | 6.95 | 7.24 | 7.58 | 7.34 | 7.30 | 7.40 | 7.38 | 7.29 |

### Example 23: ADC anti-tumor cell activity test

In the invention, MDA-MB-468 and BT474 are used as the research system of in vitro efficacy detection. An appropriate number of tumor cell were uniformly inoculated into 96-well plates and incubated in carbon dioxide incubators. 24 hours later, the cells were confirmed to be normal under the microscope, and drugs were added. The drugs were diluted by medium (the initial concentration of ADC was 500nM, the dilution factor was 7 times, there were 8 concentration points, the theoretical coupling ratio (DAR) of toxin and antibody was 8: 1, and the actual coupling ratio was roughly 7.5:1, so the initial concentration of toxin was 4.0µM, the concentration gradient dilution of 7 times, 8 concentration points). After mixing, they were added into the corresponding cell holes, and the last two columns were the control group (i.e., cell + medium, no drug treatment) and the blank group (i.e., no cells, only containing medium, for deducting background), and incubated in the carbon dioxide incubator at 37°C for 5 days. After 5 days, 20µL MTS (Promega, G3581) was added to each well for 2 h, and the absorption value of 490nm was obtained by a machine reader (from Molecular Device: SpectraMAX190). By detecting the activity of dehydrogenase in mitochondria, IC50 was calculated to evaluate the inhibitory effect of ADC drugs on tumor cell proliferation.

| **Cell Lines** | **ADC numbering** | | | | | | | | |
|---|---|---|---|---|---|---|---|---|---|
| | **ADC-1** | **ADC-2** | **ADC-3** | **ADC-5** | **ADC-6** | **ADC-7** | **ADC-8** | **ADC-9** | **ADC-10** |
| **BT474** | 14.792 | 52.21 | 3.963 | 2.787 | 1.452 | 7.486 | 1.857 | 4.109 | 8.539 |
| **MDA-MB-468** | 24.59 | 29.01 | 2.155 | 3.223 | 19.1 | 2.422 | 2.58 | 5.016 | 4.152 |

ADC-1 is the marketed antitumor drug ENHERTU^{®}. As shown in the above ADC cell activity test, the camptothecin chiral derivative drugs disclosed in the instant invention demonstrate good antitumor activity in multiple tumor cell lines after coupling with antibody through linking unit L. Compared with ADC-1, some ADCs have even higher activities, indicating great clinical application value.

### Example 24: The in vivo efficacy test of ADC

In the present invention, NCI-N87 tumor nude mouse model is established to evaluate the efficacy of ADC conjugate drug *in vivo.* In some embodiments, 5×10⁶ NCI-N87 cells were inoculated subcutaneously into the right shoulder of BALB/c nude mice aged 4-6 weeks, and the average tumor size of the mice grew to 162mm³. The mice were randomly divided according to the tumor size, with 5 mice in each group. ADC-1, ADC-2, ADC-3, ADC-5, ADC-6 and ADC-7 were administered intravenously on days 0, 7, 14 and 21 at a dose of 3mg/kg (blank buffer solution) and 3mg/kg, respectively. The tumor volume measurements were shown as the mean tumor volume at the time of measurement, and the weight changes of mice were recorded to observe the efficacy and initial toxicity of ADC drugs *in vivo.*

| **ADC numbering** | **Test content** | **0 day** | **3day** | **7day** | **10day** | **14day** | **17day** | **21day** | **24day** | **28day** | **31day** |
|---|---|---|---|---|---|---|---|---|---|---|---|
| **Blank** | Average tumor volume(mm³) | 160.54 | 226.64 | 314.71 | 421.80 | 477.89 | 574.75 | 560.01 | 727.32 | 782.74 | 778.79 |
| | Average body weight (g) | 19.91 | 20.53 | 20.75 | 20.67 | 21.41 | 22.13 | 22.44 | 21.99 | 21.97 | 22.94 |
| **Trastuzumab** | Average tumor volume(mm³) | 163.79 | 243.19 | 344.55 | 367.70 | 341.88 | 317.77 | 395.42 | 406.95 | 413.29 | 457.68 |
| | Average body weight (g) | 20.18 | 20.90 | 21.19 | 21.17 | 22.42 | 22.68 | 22.95 | 22.85 | 22.59 | 23.50 |
| **ADC-1** | Average tumor volume(mm³) | 164.40 | 177.07 | 179.04 | 166.23 | 100.80 | 137.68 | 171.61 | 197.29 | 206.69 | 249.40 |
| | Average body weight (g) | 19.51 | 20.24 | 20.78 | 21.03 | 21.99 | 21.63 | 22.23 | 22.12 | 21.95 | 22.74 |
| **ADC-2** | Average tumor volume(mm³) | 160.19 | 208.21 | 166.74 | 144.77 | 100.44 | 123.43 | 45.94 | 99.19 | 73.47 | 30.24 |
| | Average body weight (g) | 20.07 | 20.10 | 20.33 | 20.77 | 21.64 | 21.61 | 21.74 | 21.68 | 21.32 | 22.45 |
| **ADC-3** | Average tumor volume(mm³) | 160.90 | 210.46 | 230.67 | 257.95 | 212.00 | 228.95 | 172.11 | 187.24 | 160.82 | 112.08 |
| | Average body weight (g) | 20.45 | 19.91 | 20.30 | 20.65 | 21.33 | 21.14 | 21.50 | 21.59 | 21.22 | 22.47 |
| **ADC-5** | Average tumor volume(mm³) | 162.81 | 214.40 | 169.61 | 138.92 | 92.23 | 112.31 | 58.85 | 90.27 | 58.95 | 31.45 |
| | Average body weight (g) | 20.74 | 20.59 | 20.97 | 21.02 | 21.73 | 21.82 | 22.04 | 22.06 | 21.91 | 22.58 |
| **ADC-7** | Average tumor volume(mm³) | 163.29 | 184.66 | 139.76 | 98.26 | 43.92 | 64.85 | 22.76 | 35.30 | 31.30 | 13.43 |
| | Average body weight (g) | 19.88 | 20.58 | 20.59 | 20.83 | 21.81 | 21.98 | 22.23 | 22.41 | 21.97 | 22.59 |

ADC-1 is the marketed antitumor drug ENHERTU^{®}. Through the above in vivo efficacy experiment in mice, it is proved that the camptothecin chiral derivative drugs disclosed in the instant invention show clear antitumor activity in tumor bearing mice after coupling with antibody through linking unit L, and the average tumor size is significantly smaller than that of blank control. Compared with ADC-1, ADC-2 showed similar activity, ADC-5 showed stronger activity, and ADC-7 showed superior activity. The body weight of mice did not change significantly during the administration period, and no mice died in the group. The camptothecin chiral derivative drug described in the invention demonstrates excellent safety and great application value.

## Claims

1. A camptothecin derivative as shown in Formula D, or its pharmaceutically acceptable salt or solvate; 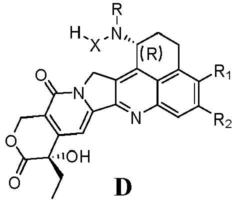 wherein:
the chiral carbon atom connected to the 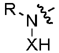 group has an absolute chirality of R configuration;
R is selected from hydrogen, alkyl, substituted alkyl, deuterated alkyl, cycloalkylalkyl, alkoxy alkyl, aryl, substituted aryl and heteroaryl;
R₁ is selected from hydrogen, deuterium, halogen, alkyl, substituted alkyl, deuterated alkyl, cycloalkylalkyl, alkoxy alkyl, carboxyl, heterocyclic, aryl, substituted aryl and heteroaryl;
R₂ is selected from hydrogen, deuterium, halogen, alkyl, substituted alkyl, deuterated alkyl, cycloalkylalkyl, alkoxy alkyl, carboxyl, heterocyclic, aryl, substituted aryl and heteroaryl;
X is selected from -C(O)-CRₐR_{b}-(CR₃R₄)ₘ-O-, -C(O)-CRₐR_{b}-(CR₃R₄)ₘ-NH-, and - C(O)-CRₐR_{b}-(CR₃R₄)ₘ-S-;
Rₐ is selected from hydrogen, deuterium, halogen, alkyl, deuterated alkyl, halogenated alkyl, cycloalkylalkyl, alkoxy alkyl, heterocyclic, aryl, substituted aryl and heteroaryl;
R_{b} is selected from hydrogen, deuterium, halogen, alkyl, deuterated alkyl, halogenated alkyl, cycloalkylalkyl, alkoxy alkyl, heterocyclic, aryl, substituted aryl and heteroaryl;
alternatively, Rₐ, R_{b} and their connected carbon atoms form a C₃₋₆ cycloalkyl, a cycloalkylalkyl or a heterocyclic group;
R₃, R₄ are identical or different, and are independently selected from hydrogen, deuterium, halogen, alkyl, haloalkyl, deuterated alkyl, alkoxy, hydroxyl, amino, cyano, nitro, hydroxyalkyl, cycloalkyl and heterocyclic group;
alternatively, R₃, R₄ and their connected carbon atoms form a C₃₋₆ cycloalkyl, a cycloalkylalkyl or a heterocyclic group; and
m is an integer from 0-4.

2. The camptothecin derivative in accordance with claim 1, or a tautomer, mesomer, racemate, enantiomer, diastereoisomer or a mixture thereof, or a pharmaceutically acceptable salt or solvate thereof, wherein R₁ is a C₁₋₃ alkyl group.

3. The camptothecin derivative in accordance with claim 1, or its tautomer, mesomer, racemate, enantiomer, diastereoisomer or a mixture thereof, or a pharmaceutically acceptable salt or solvate thereof, wherein R₂ is a C₁₋₃ alkyl or a fluorine atom.

4. The camptothecin derivative in accordance with claims 1-3, or a tautomer, mesomer, racemate, enantiomer, diastereoisomer or a mixture thereof, or a pharmaceutically acceptable salt or solvate thereof, wherein the camptothecin derivative has the structure shown in Formula D₁: 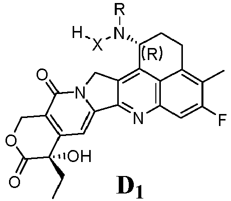 wherein:
the chiral carbon atom connected to the 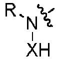 group has an absolute chirality of R configuration;
R is selected from hydrogen, deuterium, halogen, alkyl, substituted alkyl, deuterated alkyl, cycloalkylalkyl, alkoxy alkyl, aryl, substituted aryl and heteroaryl;
X is selected from -C(O)-CRₐR_{b}-(CR₃R₄)ₘ-O-,-C(O)-CRₐR_{b}-(CR₃R₄)ₘ-NH-, or -C(O)-CRₐR_{b}-(CR₃R₄)ₘ-S-;
Rₐ is selected from hydrogen, deuterium, halogen, alkyl, deuterated alkyl, halogenated alkyl, cycloalkylalkyl, alkoxy alkyl, heterocyclic, aryl, substituted aryl and heteroaryl;
R_{b} is selected from hydrogen, deuterium, halogen, alkyl, deuterated alkyl, halogenated alkyl, cycloalkylalkyl, alkoxy alkyl, heterocyclic, aryl, substituted aryl and heteroaryl;
alternatively, Rₐ, R_{b} and their connected carbon atoms form a C₃₋₆ cycloalkyl group, a cycloalkylalkyl or a heterocyclic group;
R₃, R₄ are identical or different, and are independently selected from hydrogen, deuterium, halogen, alkyl, haloalkyl, deuterated alkyl, alkoxy, hydroxyl, amino, cyano, nitro, hydroxyalkyl, cycloalkyl and heterocyclic;
alternatively, R₃, R₄ and their connected carbon atoms form a C₃₋₆ cycloalkyl group, a cycloalkylalkyl or a heterocyclic group; and
m is an integer from 0-4.

5. The camptothecin derivative in accordance with claim 4, or a tautomer, mesomer, racemate, enantiomer, diastereoisomer or a mixture thereof, or a pharmaceutically acceptable salt or solvate thereof, wherein the R is a C₁₋₃ alkyl.

6. The camptothecin derivative in accordance with claim 4, or its tautomer, mesomer, racemate, enantiomer, diastereoisomer or a mixture thereof, or a pharmaceutically acceptable salt or solvate thereof, wherein the X comprises: 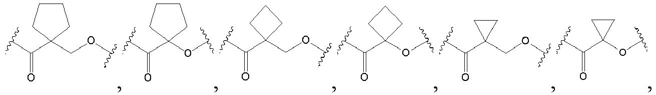 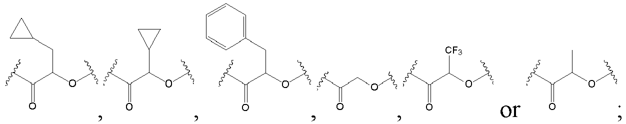 wherein the left wavy line is connected to the camptothecin derivative, and the right wavy line is connected to a linking unit.

7. The camptothecin derivative in accordance with claim 1, or a tautomer, mesomer, racemate, enantiomer, diastereoisomer or a mixture thereof, or a pharmaceutically acceptable salt or solvate thereof, comprising: 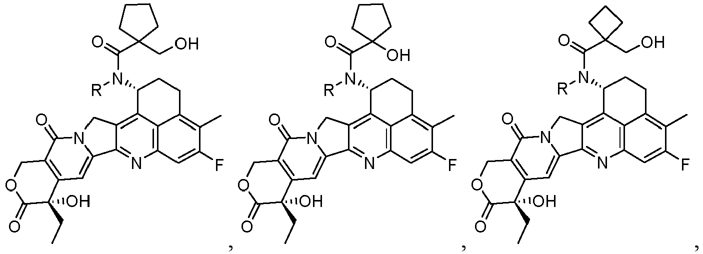 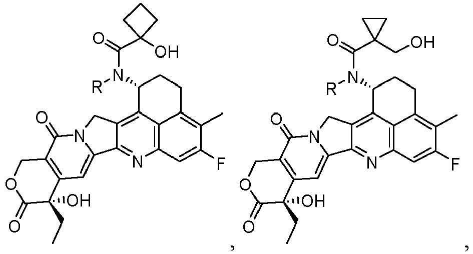 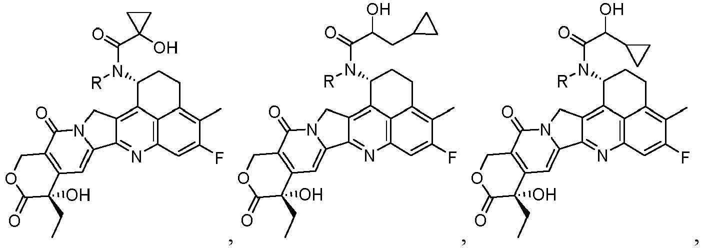 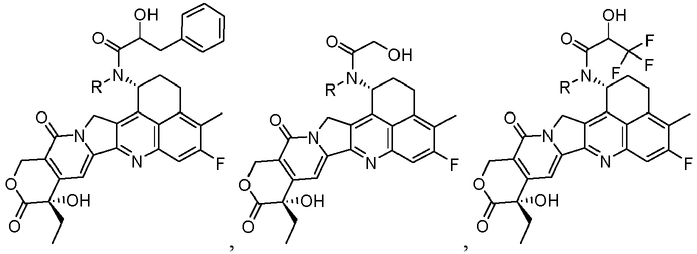 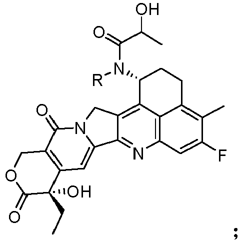 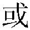 wherein R is selected from hydrogen, deuterium, halogen, alkyl, substituted alkyl, deuterated alkyl, cycloalkylalkyl, alkoxy alkyl, aryl, substituted aryl and heteroaryl.

8. The camptothecin derivative in accordance to claim 7, or its tautomer, mesomer, racemate, enantiomer, diastereoisomer or a mixture thereof, or a pharmaceutically acceptable salt or solvate thereof, comprising:

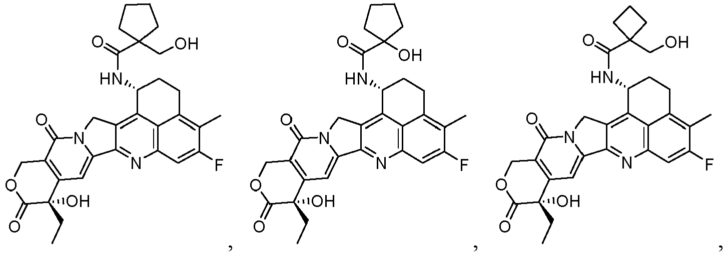

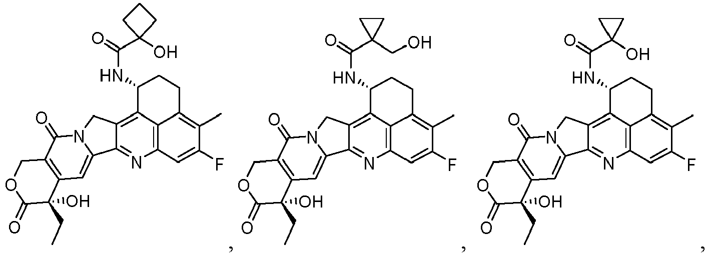

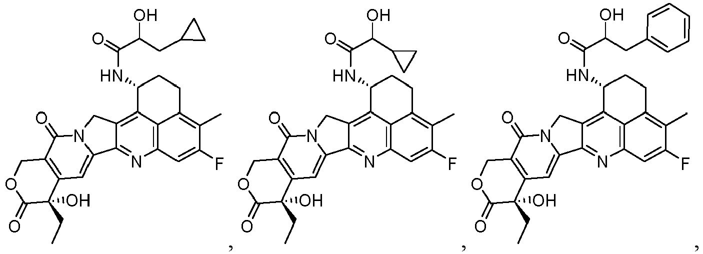

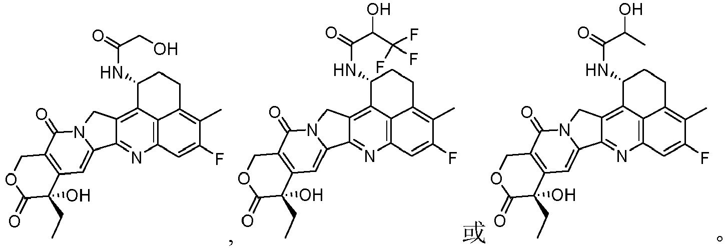

9. A linker-drug conjugate comprising the camptothecin derivative in accordance with claims 1-8 or a pharmaceutically acceptable salt or solvate thereof, or a tautomer, mesomer, racemate, enantiomer, diastereoisomer or a mixture thereof; 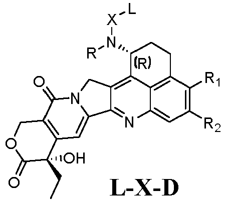 wherein:
the chiral carbon atom connected to the 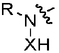 group has an absolute chirality of R configuration;
R is selected from hydrogen, alkyl, substituted alkyl, deuterated alkyl, cycloalkylalkyl, alkoxy alkyl, aryl, substituted aryl and heteroaryl;
R₁ is selected from hydrogen, deuterium, halogen, alkyl, substituted alkyl, deuterated alkyl, cycloalkylalkyl, alkoxy alkyl, carboxyl, heterocyclic, aryl, substituted aryl and heteroaryl;
R₂ is selected from hydrogen, deuterium, halogen, alkyl, substituted alkyl, deuterated alkyl, cycloalkylalkyl, alkoxy alkyl, carboxyl, heterocyclic, aryl, substituted aryl and heteroaryl;
X is selected from -C(O)-CRₐR_{b}-(CR₃R₄)ₘ-O-, -C(O)-CRₐR_{b}-(CR₃R₄)ₘ-NH-, or - C(O)-CRₐR_{b}-(CR₃R₄)ₘ-S-;
Rₐ is selected from hydrogen, deuterium, halogen, alkyl, deuterated alkyl, halogenated alkyl, cycloalkylalkyl, alkoxy alkyl, heterocyclic, aryl, substituted aryl and heteroaryl;
R_{b} is selected from hydrogen, deuterium, halogen, alkyl, deuterated alkyl, halogenated alkyl, cycloalkylalkyl, alkoxy alkyl, heterocyclic, aryl, substituted aryl and heteroaryl;
alternatively, Rₐ, R_{b} and their connected carbon atoms form a C₃₋₆ cycloalkyl group, a cycloalkylalkyl group or a heterocyclic group;
R₃, R₄ are identical or different, and are independently selected from hydrogen, deuterium, halogen, alkyl, haloalkyl, deuterated alkyl, alkoxy, hydroxyl, amino, cyano, nitro, hydroxyalkyl, cycloalkyl and heterocyclic;
alternatively, R₃, R₄ and their connected carbon atoms form a C₃₋₆ cycloalkyl group, a cycloalkylalkyl group or a heterocyclic group;
m is an integer from 0-4; and
L comprises -L₁-L₂-L₃-L₄-.

10. The linker-drug conjugate according to claim 9, or a pharmaceutically acceptable salt or solvate thereof, wherein L is -L₁-L₂-L₃-L₄-, wherein the L₁ is connected to the ligand Ab and the L₄ is connected to the X.

11. The linker-drug conjugate or a pharmaceutically acceptable salt or solvate thereof in accordance with claims 9 or 10, wherein the L₁ comprises:

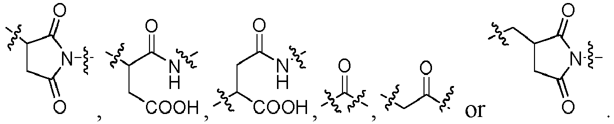

12. The linker-drug conjugate according to claims 9 or 10, or a pharmaceutically acceptable salt or solvate thereof, wherein:
L₂ is selected from -NC(R₅R₆)C(O), -NR₇(CH₂)ₒC(O)-, -NR₇(CH₂CH₂O)ₒCH₂C(O)-, - S(CH₂)ₚC(O)- or a chemical bond, wherein o is an integer from 0-20, p is an integer from 0-20;
R₅ and R₆ are identical or different, and independently selected from hydrogen, deuterium, alkyl, substituted alkyl, deuterated alkyl, heteroalkyl, carboxyl, amino, and substituted amino;
R₇ is selected from hydrogen, deuterium, halogen, alkyl, substituted alkyl, deuterated alkyl, cycloalkylalkyl alkyl, alkoxy alkyl, aryl, substituted aryl and heteroaryl; and
L₁ and L₂ use a common N atom.

13. The linker-drug conjugate according to claims 9 or 10, or a pharmaceutically acceptable salt or solvate thereof, wherein L₃ is a peptide residue comprising amino acids, wherein one or more amino acid is substituted by or one or more substituents selected from deuterium atom, halogen, hydroxyl, cyano, amino, nitro, carboxyl, alkyl, substituted alkyl, alkoxy, cycloalkyl, and substituted cycloalkyl.

14. The linker-drug conjugate according to claims 9 or 10, or a pharmaceutically acceptable salt or solvate thereof wherein L₃ preferably comprises a peptide residue that comprises 1, 2 or more amino acids selected from phenylalanine (F), glycine (G), valine (V), lysine (K), citrulline, serine (S), glutamic acid (E), and aspartic acid (D).

15. The linker-drug conjugate according to claim 9 or 10, or a pharmaceutically acceptable salt or solvate thereof, wherein:
L₄ is selected from: NR₈(CR₉R₁₀)_{q}-, -C(O)NR₈-, -C(O)NR₈(CH₂)_{q}- or a chemical bond, q is selected from integers 0-6; and
R₈, R₉ and R₁₀ are identical or different, and independently selected from hydrogen, deuterium, halogen, alkyl, substituted alkyl, deuterated alkyl, cycloalkylalkyl, alkoxy alkyl, heterocyclic, aryl, substituted aryl and heteroaryl.

16. The linker-drug conjugate according to claim 9, or a pharmaceutically acceptable salt or solvate thereof, wherein the linker L comprises; 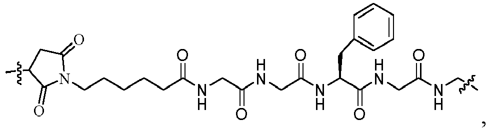 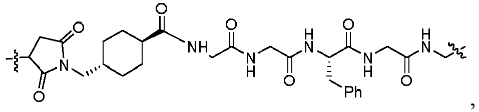 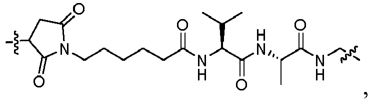 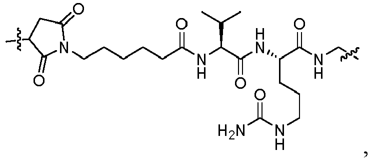 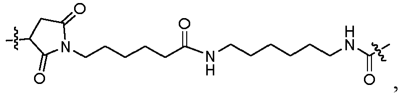 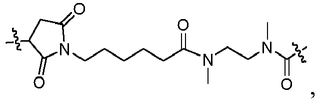 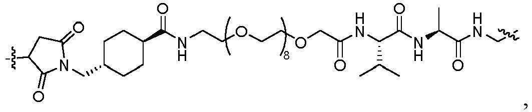 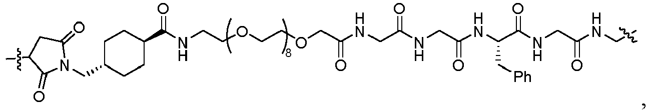 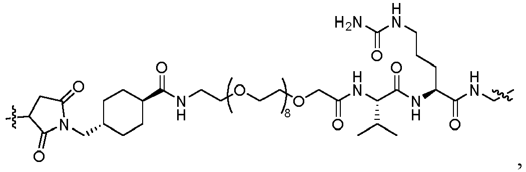 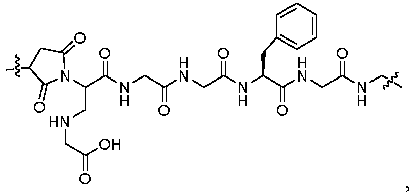 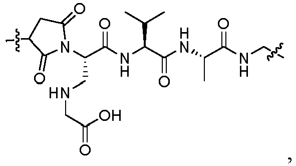 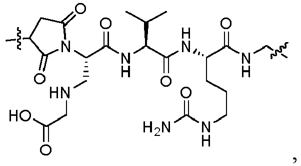 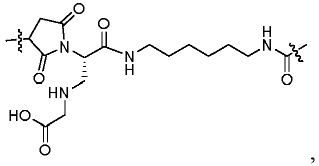 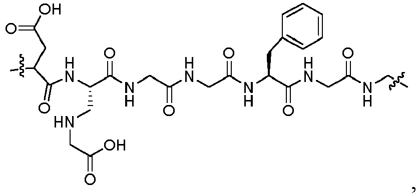 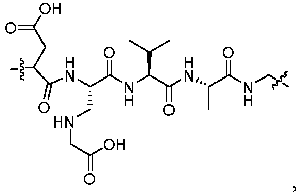 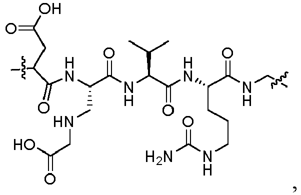 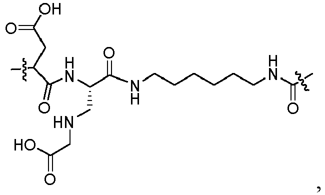 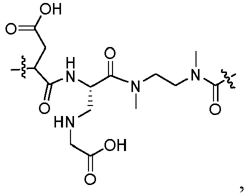 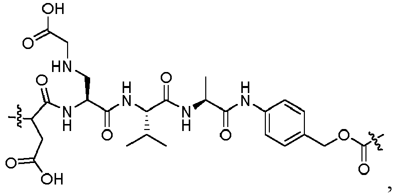 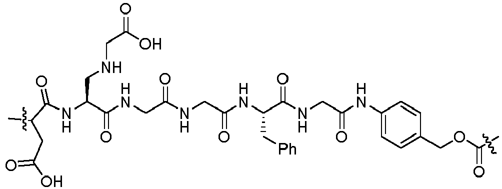 or 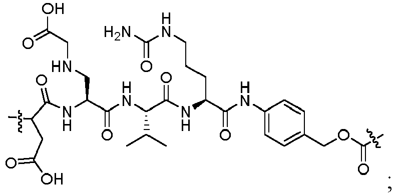 wherein the left wavy line is connected to a ligand, and the right wavy line is connected to the X.

17. The linker-drug conjugate according to claim 16, or a pharmaceutically acceptable salt or solvate thereof, wherein the linker-drug conjugate comprises: 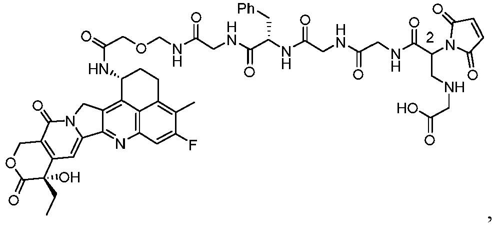 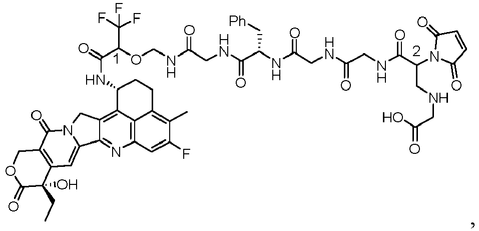 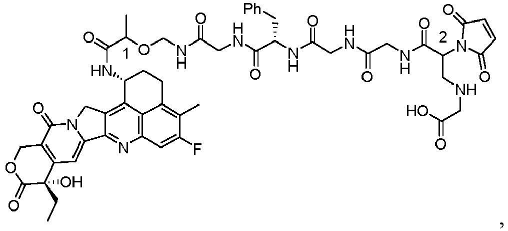 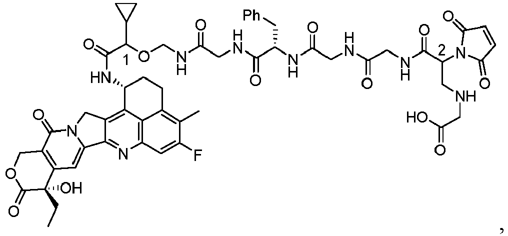 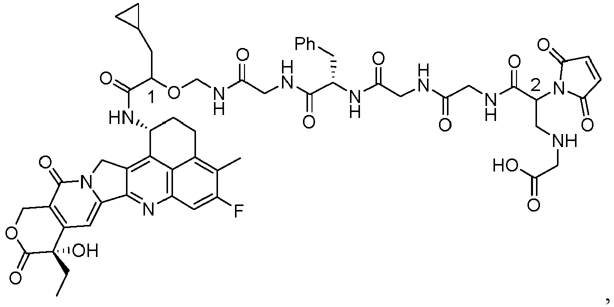 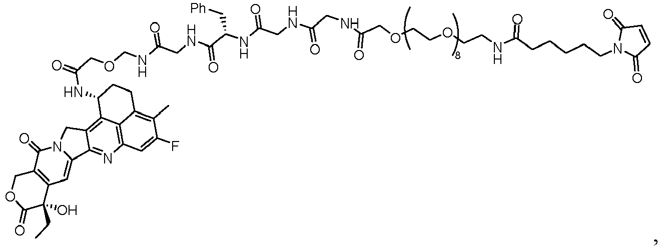 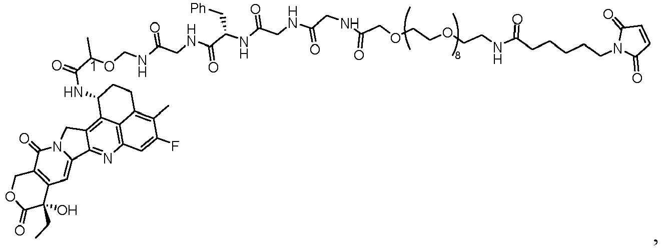 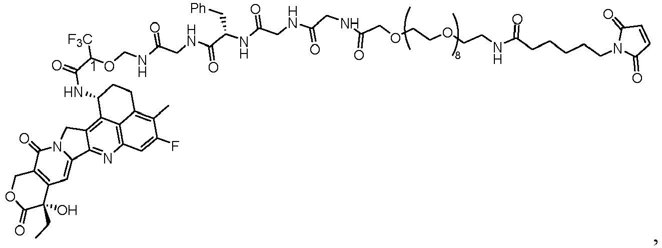 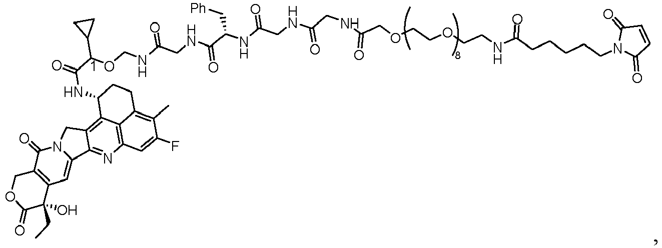 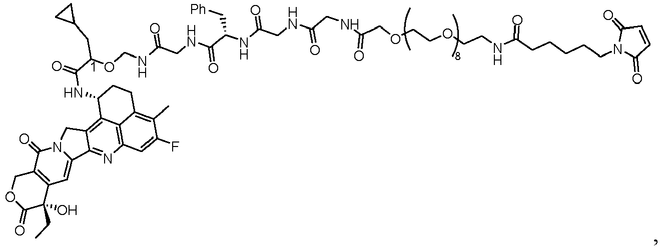 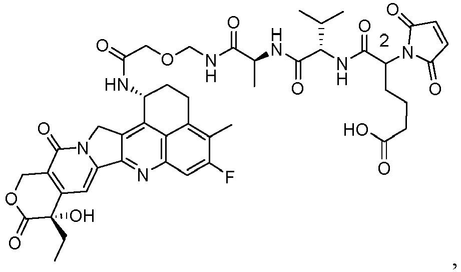 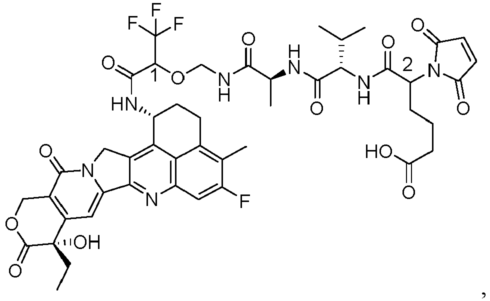 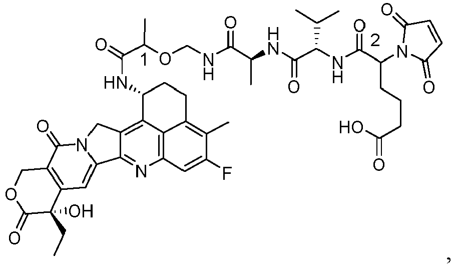 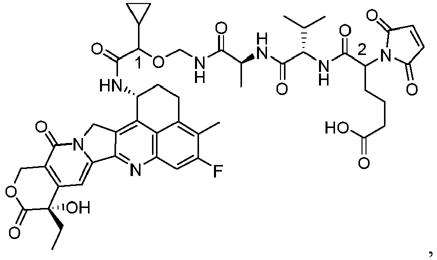 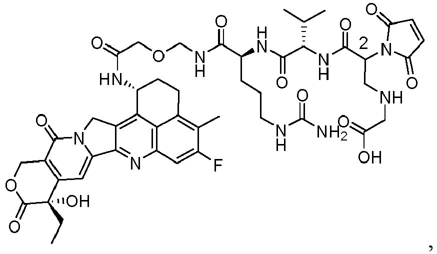 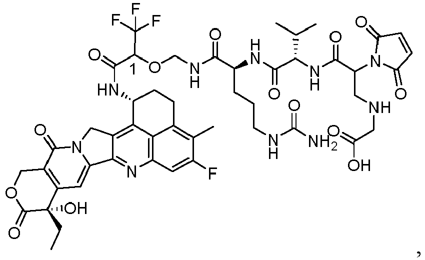 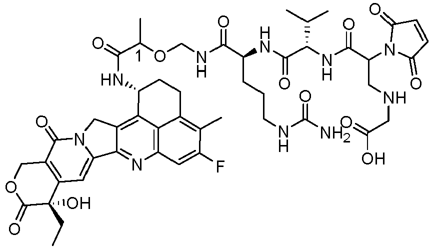 or 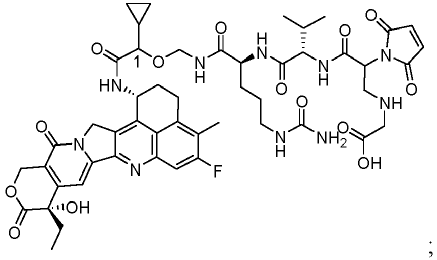 wherein:
the carbon atom at position 1 has an absolute chirality of either R or S configuration.

18. A ligand-drug conjugate according to claims 9-17, or a pharmaceutically acceptable salt or solvate thereof, comprising the linker-drug conjugate, wherein the ligand-drug conjugate comprises the structure of Formula I: 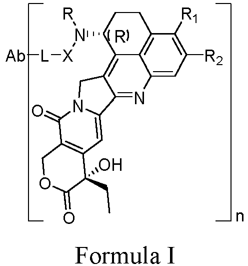 wherein:
the chiral carbon atom connected to the 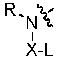 group has an absolute chirality of R configuration;
R is selected from hydrogen, deuterium, halogen, alkyl, substituted alkyl, deuterated alkyl, cycloalkylalkyl, alkoxy alkyl, aryl, substituted aryl and heteroaryl;
R₁ is selected from hydrogen, deuterium, halogen, alkyl, substituted alkyl, deuterated alkyl, cycloalkylalkyl, alkoxy alkyl, carboxyl, heterocyclic, aryl, substituted aryl and heteroaryl;
R₂ is selected from hydrogen, deuterium, halogen, alkyl, substituted alkyl, deuterated alkyl, cycloalkylalkyl, alkoxy alkyl, carboxyl, heterocyclic, aryl, substituted aryl and heteroaryl;
X is selected from -C(O)-CRₐR_{b}-(CR₃R₄)ₘ-O-, -C(O)-CRₐR_{b}-(CR₃R₄)ₘ-NH- or - C(O)-CRₐR_{b}-(CR₃R₄)ₘ-S-;
Rₐ is selected from hydrogen, deuterium, halogen, alkyl, deuterated alkyl, halogenated alkyl, cycloalkylalkyl, alkoxy alkyl, heterocyclic, aryl, substituted aryl and heteroaryl;
R_{b} is selected from hydrogen, deuterium, halogen, alkyl, deuterated alkyl, halogenated alkyl, cycloalkylalkyl, alkoxy alkyl, heterocyclic, aryl, substituted aryl and heteroaryl;
alternatively, Rₐ, R_{b} and their connected carbon atoms form a C₃₋₆ cycloalkyl, a cycloalkylalkyl or a heterocyclic group;
R₃, R₄ are identical or different, and are independently selected from hydrogen, deuterium, halogen, alkyl, haloalkyl, deuterated alkyl, alkoxy, hydroxyl, amino, cyano, nitro, hydroxyalkyl, cycloalkyl and heterocyclic;
alternatively, R₃, R₄ and their connected carbon atoms form a C₃₋₆ cycloalkyl group, a cycloalkylalkyl or a heterocyclic group;
Ab is a ligand unit selected from antibodies, antibody fragments, targeted proteins, Fc-fusion proteins, etc.;
L is an Ab linking unit; X is a drug modification unit; and
m is an integer from 0-4; n is an integer or a decimal between 1 and 20.

19. The ligand-drug conjugate according to claim 18, or a pharmaceutically acceptable salt or solvate thereof, wherein the Ab is an antibody having a linking bond with a linking unit through its heteroatom, wherein the Ab is selected from murine antibodies, chimeric antibodies, humanized antibodies, fully human antibodies, antibody fragments, bispecific antibodies and multispecific antibodies.

20. The ligand-drug conjugate according to claim 19, or a pharmaceutically acceptable salt or solvate thereof, wherein the antibody or an antigen-binding fragment comprises anti-EGFRvIII antibody, anti-DLL-3 antibody, anti-PSMA antibody, anti-CD70 antibody, anti-MUC16 antibody, anti-ENPP3 antibody, anti-TDGF1 antibody, anti-ETBR antibody, anti-MSLN antibody, anti-TIM-1 antibody, anti-LRRC15 antibody, Anti-LIV-1 antibody, anti-CanAg/AFP antibody, anti-cladin 18.2 antibody, anti-Mesothelin antibody, anti-HER2 (ErbB2) antibody, anti-EGFR antibody, anti-c-MET antibody, anti-SLITRK6 antibody, anti-KIT/CD117 antibody, anti-STEAP1 antibody, anti-SLAMF7/CS1 antibody, anti-NaPi2B/SLC34A2 antibody, anti-GPNMB antibody, anti-HER3 ( ErbB3) antibody, anti-MUC1/CD227 antibody, anti-AXL antibody, anti-CD166 antibody, anti-B7-H3 (CD276) antibody, anti-PTK7/CCK4 antibody, anti-PRLR antibody, anti-EFNA4 antibody, anti-5T4 antibody, anti-NOTCH3 antibody, anti-Nectin 4 antibody, anti-TROP-2 antibody, anti-CD142 antibody, anti-CA6 antibody, anti-GPR20 antibody, anti-CD174 antibody, Anti-CD71 antibody, anti-EphA2 antibody, anti-LYPD3 antibody, anti-FGFR2 antibody, anti-FGFR3 antibody, anti-FRα antibody, anti-CEACAMs antibody, anti-GCC antibody, anti-Integrin Av antibody, anti-CAIX antibody, anti-P-cadherin antibody, anti-GD3 antibody, anti-Cadherin 6 antibody, anti-LAMP1 antibody, anti-FLT3 antibody, anti-BCMA antibody, anti-CD79b antibody, anti-CD19 antibody , anti-CD33 antibody, anti-CD56 antibody, anti-CD74 antibody, anti-CD22 antibody, anti-CD30 antibody, anti-CD37 antibody, anti-CD47 antibody, anti-CD138 antibody, anti-CD352 antibody, anti-CD25 antibody and anti-CD123 antibody.

21. The ligand-drug conjugate according to claims 19 or 20, or its pharmaceutically acceptable salt or solvate, or a tautomer, racemate, racemate, enantiomer, enantiomer or mixture thereof, wherein the ligand-drug conjugate comprises: 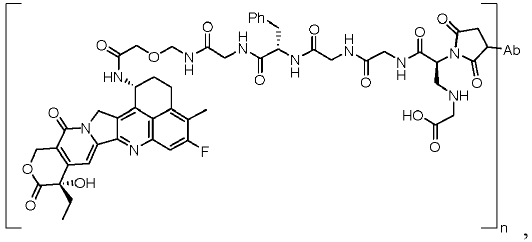 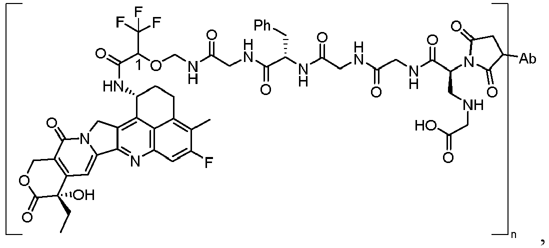 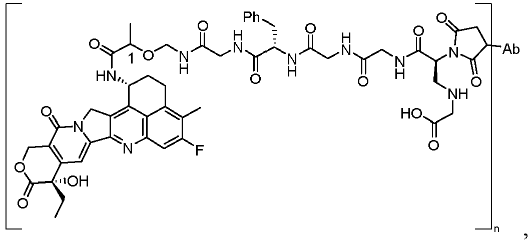 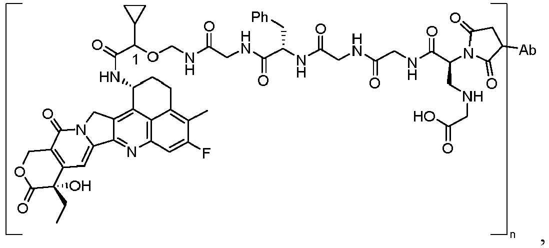 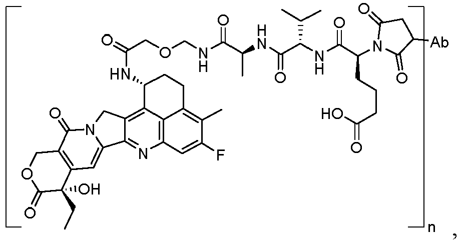 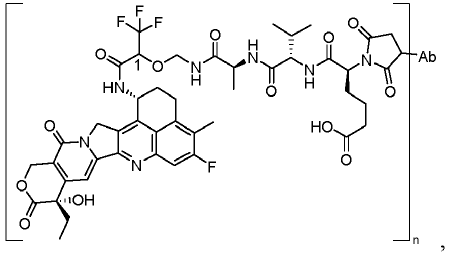 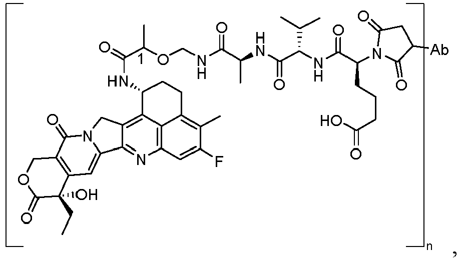 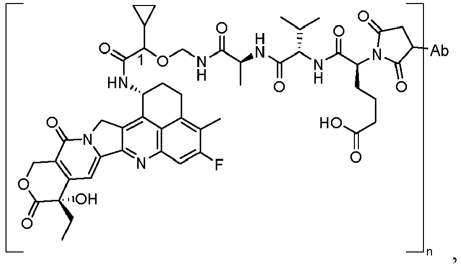 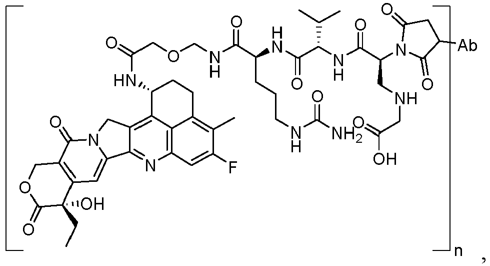 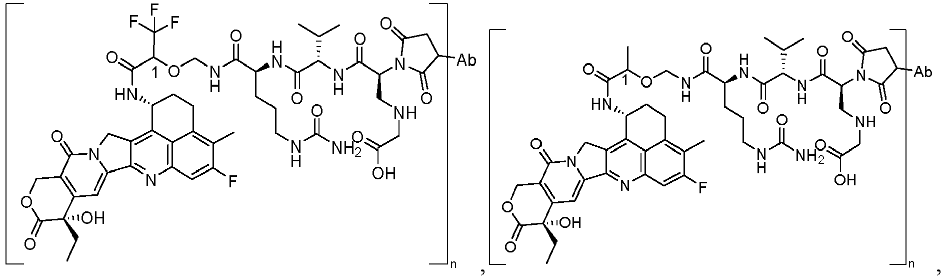 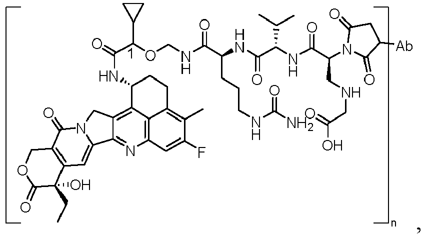 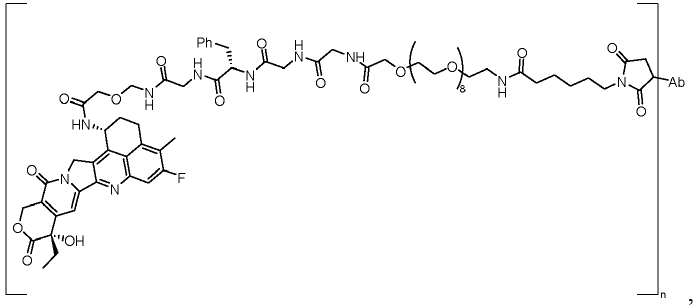 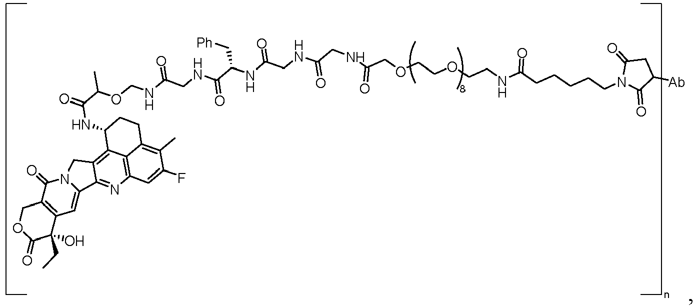 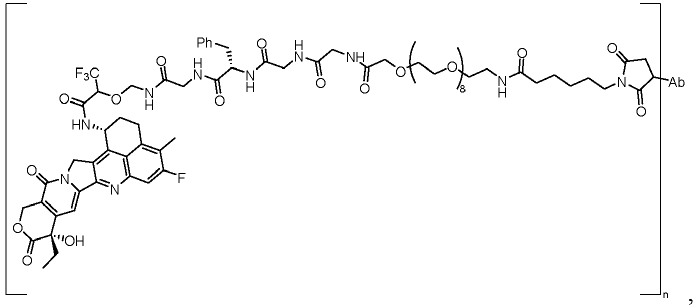 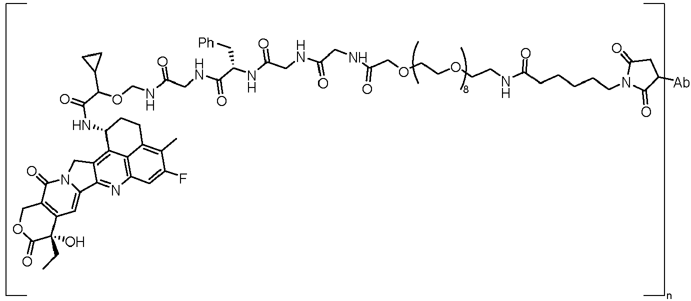 or 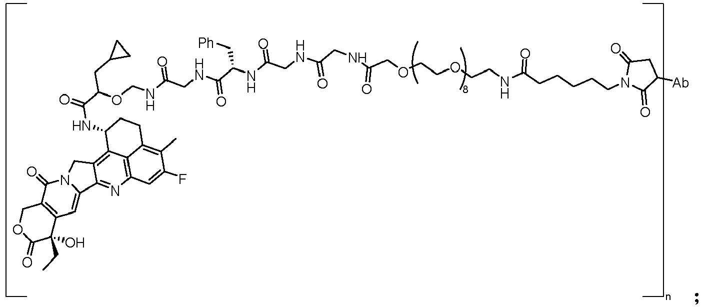 wherein:
the Ab is the ligand unit; n is an integer or a decimal between 1-20; and
the carbon atom of the position 1 has an absolute chirality of either R or S configuration.

22. A method for preparing any of the linker-drug conjugates of claims 9-17, or their tautomers, mesomers, racemates, enantiomers, diastereoisomers or a mixture thereof, or pharmaceutically acceptable salts or solvates thereof, comprising the following steps: 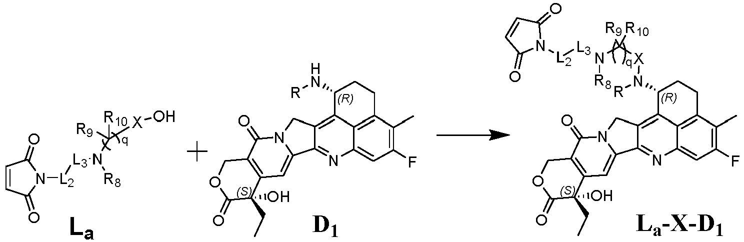
through a replacement reaction between the linking unit La and the compound of Formula D₁, obtaining the linker-drug conjugate of Formula Lₐ-X-D₁;
wherein:
the chiral carbon atom connected to the 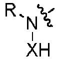 group has an absolute chirality of R configuration;
L₂, L₃, R, R₈, R₉, R₁₀, q and X are described in Formula L-X-D.

23. A method for preparing the ligand-drug conjugate or its tautomers, racemates, racemates, enantiomers, enantiomers or mixtures thereof or a pharmaceutically acceptable salt or solvate thereof of claims 18-21, further comprising the following steps: 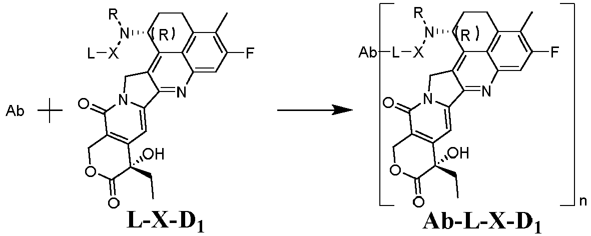
conjugating a reduced antibody, antibody fragment or antigen-binding fragment with the linker-drug conjugate of Formula L-X-D₁ to obtain the ligand-drug conjugate as shown in Formula Ab-L-X-D₁
wherein:
the chiral carbon atom connected to the 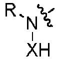 group has an absolute chirality of R configuration; and
Ab, L, X, R and n are described in Formula I.

24. The camptothecin derivative, linker-drug conjugate or ligand-drug conjugate, or a pharmaceutically acceptable salt or solvate thereof, all according to claims 1-23, wherein the pharmaceutically acceptable salt comprises sodium, potassium, calcium or magnesium salts formed with acidic functional groups in the structure camptothecin derivative, linker-drug conjugate or ligand-drug conjugate; or acetate, trifluoroacetate, citrate, oxalate, tartrate, malate, nitrate, chloride, bromide, iodide, sulfate, bisulfate, phosphate, lactate, oleate, ascorbate, salicylate, formate, glutamate, methanesulfonate, ethanesulfonate, benzenesulfonate or p-toluenesulfonate formed with basic functional groups in the structure of camptothecin derivative, linker-drug conjugate or ligand-drug conjugate.

25. A pharmaceutical composition comprising a therapeutically effective amount of the camptothecin derivative, linker-drug conjugate or ligand-drug conjugate of claims 1-24, or its tautomers, mesomers, racemates, enantiomers, diastereoisomers or a mixture thereof, or pharmaceutically acceptable salt or solvate thereof, and a pharmaceutically acceptable carrier, diluent or excipient.

26. The use, for purpose of preparing drugs for the treatment or prevention of tumor, of the camptothecin derivative, linker-drug conjugate or ligand-drug conjugate of claims 1-24, or their tautomers, mesomers, racemates, enantiomers, diastereoisomers or a mixture thereof, or a pharmaceutically acceptable salt or solvent compound thereof, and a pharmaceutically acceptable carrier, diluent, or excipient.

27. The use according to claim 26, wherein the tumor comprises solid tumor and hematological tumor.

28. The use according to claim 27, wherein the tumor comprises breast, ovarian, cervical, uterine, prostate, kidney, urethral, bladder, liver, gastric, endometrium, salivary gland cancer, esophageal cancer, lung cancer, colon cancer, rectal cancer, colorectal cancer, bone cancer, skin cancer, thyroid cancer, pancreatic cancer, melanoma, glioma, neuroblastoma, glioblastoma, sarcoma, lymphoma or leukemia.
